(19) Europäisches Patentamt
European Patent Office
Office européen des brevets

(11) Publication number: **0 254 737 B1**

(12) **EUROPEAN PATENT SPECIFICATION**

(45) Date of publication of patent specification: **25.07.90**

(51) Int. Cl.[5]: **C 07 D 223/14,** C 07 D 223/32, C 07 D 491/052, C 07 D 495/22, A 61 K 31/55 // (C07D491/052, 311:00, 223:00),(C07D495/22, 335:00, 223:00)

(21) Application number: **87900939.7**

(22) Date of filing: **14.01.87**

(86) International application number: **PCT/US87/00042**

(87) International publication number: **WO 87/04430 30.07.87 Gazette 87/17**

(54) **FUSED BENZAZEPINES.**

(30) Priority: **16.01.86 US 820471**

(43) Date of publication of application: **03.02.88 Bulletin 88/05**

(45) Publication of the grant of the patent: **25.07.90 Bulletin 90/30**

(84) Designated Contracting States: **AT BE CH DE FR GB IT LI LU NL SE**

(56) References cited:
**FR-A-2 137 626**
**GB-A-1 221 324**
**US-A-3 393 192**
**US-A-3 939 165**

**Chemical & Pharmaceutical Bulletin, Vol. 32(1984), pages 130-151**

**The file contains technical information submitted after the application was filed and not included in this specification**

(73) Proprietor: **SCHERING CORPORATION**
**2000 Galloping Hill Road**
**Kenilworth New Jersey 07033 (US)**

(72) Inventor: **BERGER, Joel, Gilbert**
**50 West Lindsley Road**
**Cedar Grove, NJ 07009 (US)**
Inventor: **CHANG, Wei, Kong**
**63 West Cedar Street**
**Livingston, NJ 07039 (US)**
Inventor: **GOLD, Elijah, Herman**
**10 Roosevelt Avenue**
**West Orange, NJ 07052 (US)**
Inventor: **CLADER, John, Welch**
**25 Stratford Terrace**
**Cranford, NJ 07016 (US)**

(74) Representative: **von Kreisler, Alek, Dipl.-Chem. et al**
**Deichmannhaus am Hauptbahnhof**
**D-5000 Köln 1 (DE)**

**EP 0 254 737 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to fused derivatives of compounds having a fused ring nucleus which includes a 2,3,4,5-tetrahydro-1*H*-3-benzazepine system, to methods for their preparation, to intermediates useful in their preparation, and to pharmaceutical compositions containing them. The compounds have valuable pharmaceutical properties in the treatment of psychoses, depression, pain and hypertension.

Substituted 1-phenyl-2,3,4,5-tetrahydro-1*H*-3-benzazepines have been described in the art. For example, see U.S. Patents 3,393,192, 3,609,138, 4,011,319, 4,284,555 and 4,477,378 as well as British Patent 1,118,688. The activities discussed for the compounds disclosed in these patents include antibacterial effects, central nervous system effects and hypotensive effects.

US Patent 3,939,165 discloses 5,6,6a,6b,7,8-hexahydrobenz[a]phenanthridine hydrochloride which possesses pharmacological activity as an antidepressant. This compound has four fused six membered rings one of which contains nitrogen.

This invention relates to compounds according to the structural formula I, including all isomers and pharmaceutically acceptable salts thereof,

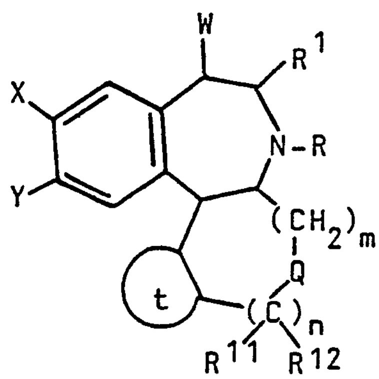

wherein:

R is hydrogen, $C_1$—$C_6$-alkyl, —$CH_2CH{=}CH_2$ or

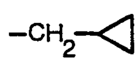 ;

$R^1$, $R^{11}$ and $R^{12}$ are the same or different and each is hydrogen or $C_1$—$C_6$-alkyl;

Q is methylene, —O— or —S—;

m and n are independently variable and may each have a value of 0, 1 or 2, with the provisos that the sum of m and n is not greater than 3, that m may not equal zero when Q is —O— or —S—, and that when Q is —$CH_2$— m and n cannot both be zero, unless W is H, X is $OCH_3$, Y is OH, Z is H and R is $CH_3$;

X is hydrogen, F, Cl, Br, I, $C_1$—$C_6$-alkyl, $C_1$—$C_6$-alkylthio, $C_1$—$C_6$-alkylsulfinyl, $C_1$—$C_6$-alkylsulfonyl, hydroxy, $C_1$—$C_6$-alkoxy or trifluoromethyl;

Y is hydrogen, hydroxy, $C_1$—$C_6$-alkoxy,

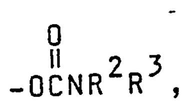 , 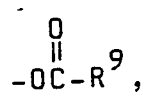 ,

$-NR^1_2$, 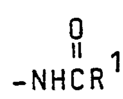 or

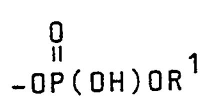 ;

where

$R^1$ is as defined above;

W is hydrogen, hydroxy or $C_1$—$C_6$-alkoxy;

ring

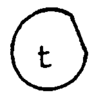

represents a fused thiophene or fused benzene ring said fused benzene ring optionally being substituted with a substituent Z as defined below;

$R^2$ and $R^3$ are independently hydrogen (provided that both are not hydrogen, $C_1$—$C_6$-alkyl, phenyl-$C_1$—$C_6$-alkyl, cyclo-$C_2$—$C_7$-alkyl, phenyl or phenyl monosubstituted by $C_1$—$C_6$-alkyl, hydroxy, $C_1$—$C_6$-alkoxy, F, Cl, Br, I or trifluormethyl, hydroxy-$C_1$—$C_6$-alkyl, or $C_1$—$C_6$-alkoxy-$C_1$—$C_6$-alkyl;

in addition, when one of $R^2$ and $R^3$ is as defined above, the other may be —$R^4NR^5R^6$ { whereinR$^4$ is $C_1$—$C_6$-alkanediyl, $R^5$ is hydrogen or $C_1$—$C_6$-alkyl and $R^6$ is $C_1$—$C_6$-alkyl, or $R^5$ and $R^6$ together with the nitrogen atom form a 1-azetidinyl, 1-pyrrolidinyl, 1-piperidinyl, 1-(4-$C_1$—$C_6$-(alkylpiperazinyl), 4-morpholinyl or 1-hexahydroazepinyl group},

in further addition, $R^2$ and $R^3$ together with the nitrogen atom may form a 1-azetidinyl, 1-pyrrolidinyl, 1-piperidinyl, 4-morpholinyl, 1-(4-($C_1$—$C_6$)-alkylpiperazinyl), 1-(4-($C_1$—$C_6$)-alkoxy-($C_1$—$C_6$)-alkylpiperazinyl), 1-(4-hydroxy-($C_1$—$C_6$)-alkylpiperazinyl), 1-(3-hydroxyazetidinyl), 1-(3-($C_1$—$C_6$)-alkoxyazetidinyl), 1-(3-hydroxy-pyrrolidinyl), 1-(3-($C_1$—$C_6$)-alkoxypyrrolidinyl), 1-(3- or 4-hydroxypiperidinyl), 1-($C_1$—$C_6$-) (3- or 4-($C_1$—$C_6$)-alkoxypiperidinyl), 1-(4-oxopiperidinyl) or 1-(3-oxopyrrolidinyl) ring;

in still further addition, when $R^2$ is hydrogen, $R^3$ may be —$CHR^7CO_2R^8$, wherein $R^7$ and $R^8$ are independently hydrogen, $C_1$—$C_6$-alkyl or phenyl-$C_1$—$C_6$-alkyl;

$R^9$ is $C_1$—$C_6$-alkyl, phenyl-$C_1$—$C_6$-alkyl, phenyl or phenyl monosubstituted by $C_1$—$C_6$-alkyl, hydroxy, $C_1$—$C_6$-alkoxy, F, Cl, Br, I or trifluormethyl, $C_1$—$C_6$-alkoxy-$C_1$—$C_6$-alkyl, phenyloxy-$C_1$—$C_6$-alkyl, phenyl-$C_1$—$C_6$-alkoxy-$C_1$—$C_6$-alkyl, cyclo-$C_3$—$C_7$-alkyl-$C_1$—$C_6$-alkyl, $C_1$—$C_6$-alkoxycarbonyl-$C_1$—$C_6$-alkyl, cyclo-$C_3$—$C_7$-alkyl, 1-adamantyl, cyclo-$C_3$—$C_7$-alkoxy-$C_1$—$C_6$-alkyl, $C_1$—$C_6$-alkoxy, phenyl-$C_1$—$C_6$-alkoxy, cyclo-$C_1$—$C_6$-alkoxy, phenoxy or phenoxy monosubstituted by $C_1$—$C_6$-alkyl, hydroxy, $C_1$—$C_6$-alkoxy, F, Cl, Br, I or trifluormethyl or —$CHR^7NHR^8$ {wherein $R^7$ and $R^8$ are as defined above}; and

Z is X as defined above, amino, $C_1$—$C_6$-alkylamino or

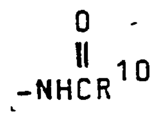

{wherein $R^{10}$ is hydrogen, $C_1$—$C_6$-alkyl or phenyl or phenyl monosubstituted by $C_1$—$C_6$-alkyl, hydroxy, $C_1$—$C_6$-alkoxy, F, Cl, Br, I or trifluormethyl}.

A preferred subgenus is represented by structural formula Ia below:

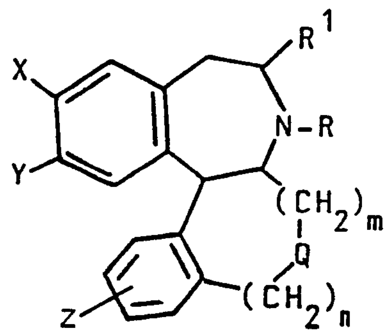

Ia

wherein R, $R^1$, X, Y, Z, Q, m and n are as defined above.

A second preferred subgenus of formula I is wherein Y is

$$-O-\overset{O}{\overset{\|}{C}}NR^2R^3,$$

(wherein $R^2$ and $R^3$ are both $C_1$—$C_6$-alkyl or one of $R^2$ and $R^3$ is hydrogen and the other is $C_1$—$C_6$-alkyl), —$NHR^1$ (wherein $R^1$ is hydrogen or methyl),

$$NH\overset{O}{\overset{\|}{C}}R^1$$

(wherein $R^1$ is hydrogen or methyl),

$$-O\overset{O}{\overset{\|}{C}}R^9$$

(wherein $R^9$ is as defined above) amino or hydroxy. W is preferably H. X is hydrogen, $C_1$—$C_6$-alkyl, F, Cl, Br, I or $C_1$—$C_6$-alkoxy; while Z is hydrogen, F, Cl, Br, I, $C_1$—$C_6$-alkyl, hydroxy or $C_1$—$C_6$-alkoxy. R is methyl and $R^1$ is hydrogen or methyl. Ring

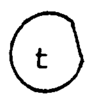

represents a fused benzene ring optionally substituted with F, Cl, Br, I, $C_1$—$C_6$-alkyl, or —$OR^1$.

A third preferred subgenus of compounds is those of formula Ia above wherein R is methyl; $R^1$ is hydrogen; Q is methylene; the sum of m and n equals 1; X is hydrogen, methyl, methoxy, chloro or bromo; Y is hydroxy, amino,

$$-O\overset{O}{\overset{\|}{C}}R^9,$$

(wherein $R^9$ is defined as above,

$$-O\overset{O}{\overset{\|}{C}}N(CH_3)_2$$

or —$NHCH_3$; and Z is hydrogen, halo, $C_1$—$C_6$-alkyl or —$OR^1$ (wherein $R^1$ is hydrogen or $C_1$—$C_6$-alkyl); or a pharmaceutically acceptable salt of such a compound.

Preferred compounds of formula I include

(1) 6,7,7a,8,9,13b-hexahydro-2-hydroxy-3-methoxy-7-methyl-5*H*-benzo[d]naphtho[2,1-b]azepine or a pharmaceutically acceptable salt thereof;

(2) 6,7,7a,8,9,13b-hexahydro-2-hydroxy-7-methyl-5*H*-benzo[d]naphtho[2,1-b]azepine or a pharmaceutically acceptable salt thereof;

(3) 6,7,7a,8,9,13b-hexahydro-3-chloro-2-hydroxy-7-methyl-5*H*-benzo[d]naphtho[2,1-b]azepine or a pharmaceutically acceptable salt thereof;

(4) 6,7,7a,8,9,13b-hexahydro-2-hydroxy-3,7-dimethyl-5*H*-benzo[d]naphtho[2,1-b]azepine or a pharmaceutically acceptable salt thereof;

(5) 6,7,7a,8,9,13b-hexahydro-2-amino-7-methyl-5*H*-benzo[d]naphtho[2,1-b]azepine or a pharmaceutically acceptable salt thereof;

(6) 6,7,7a,8,9,13b-hexahydro-2-amino-3-chloro-7-methyl-5*H*-benzo[d]naphtho[2,1-b]azepine or a pharmaceutically acceptable salt thereof;

(7) 6,7,7a,8,9,13b-hexahydro-2-amino-3,7-dimethyl-5*H*-benzo[d]naphtho[2,1-b]azepine or a pharmaceutically acceptable salt thereof;

(8) 6,6a,7,8,9,13b-hexahydro-12-methoxy-7-methyl-[1]benzopyrano[4,3-a][3]benzazepine or a pharmaceutically acceptable salt thereof; or

(9) 6,6a,7,8,9,13b-hexahydro-3-hydroxy-2-methoxy-7-methyl-5*H*-benzo[d]naphtho[2,1-b]azepine or a pharmaceutically acceptable salt thereof; or

(10) 2-hydroxy-3-methoxy-7-methyl-5,6,7,7a,8,9,10,14b-octahydro-benzo[d]benzo[3,4]cyclohepta[1,2-b]azepine or a pharmaceutically acceptable salt thereof; and

(11) 3-hydroxy-3-methoxy-7-methyl-5,6,7,7a,8,9,10,14b-octahydro-benzo[d]benzo[3,4]cyclohepta[1,2-b]azepine; geometrical isomers or a pharmaceutically acceptable salt thereof.

Particularly preferred compounds are

trans-6,7,7a,8,9,13b-hexahydro-3-chloro-2-hydroxy-7-methyl-5H-benzo[d]naphtho[2,1-b]azepine or a pharmaceutically acceptable salt thereof;

(−)-trans-6,7,7a,8,9,13b-hexahydro-3-chloro-2-hydroxy-7-methyl-5*H*-benzo[d]naphtho[2,1-b]azepine or a pharmaceutically acceptable salt thereof;

(+)-trans-6,7,7a,8,9,13b-hexahydro-3-chloro-2-hydroxy-7-methyl-5H-benzo[d]naphtho[2,1-b]azepine or a pharmaceutically acceptable salt thereof;

trans-6,7,7a,8,9,13b-hexahydro-2-hydroxy-3-methoxy-7-methyl-5H-benzo[d]naphtho[2,1-b]azepine or a pharmaceutically acceptable salt thereof; and,

trans-6,6a,7,8,9,13b-hexahydro-12-hydroxy-7-methyl-[1]benzopyrano[4,3-a][3]benzazepine or a pharmaceutically acceptable salt thereof.

Another aspect of the invention is a pharmaceutical composition which comprises a compound having structural formula I as defined in combination with a pharmaceutically acceptable carrier.

Another aspect of the invention is the use of a compound of formula I for the preparation of a pharmaceutical composition useful in the treatment of psychoses, pain and/or depression.

Yet another aspect of the invention comprises a method of making a pharmaceutical composition comprising mixing a compound of formula I with a pharmaceutically acceptable carrier.

Still another aspect of the invention comprises intermediate compounds having the formulae XI and XIV

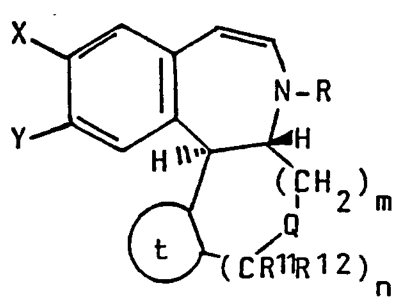

XI

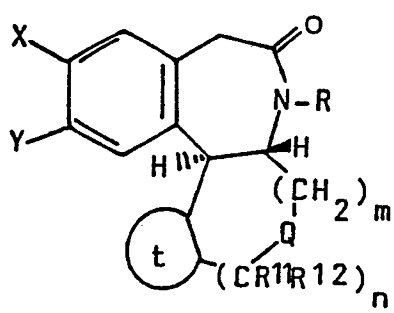

XIV

useful in the preparation of compounds of formula I wherein:

R is hydrogen, $C_1$—$C_6$-alkyl, —$CH_2CH{=}CH_2$ or

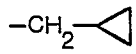 ;

$R^1$, $R^{11}$ and $R^{12}$ are the same or different and each is hydrogen or $C_1$—$C_6$-alkyl;

Q is methylene, —O— or —S—;

m and n are independently variable and may each have a value of 0, 1 or 2, with the provisos that the sum of m and n is not greater than 3, that m may not equal zero when Q is —O— or —S—, and that when Q is —$CH_2$— m and n cannot both be zero, unless W is H, X is $OCH_3$, Y is OH, B is H, $R^1$ is H and R is $CH_3$;

X is hydrogen, F, Cl, Br, I, $C_1$—$C_6$-alkyl, $C_1$—$C_6$-alkylthio, $C_1$—$C_6$-alkylsulfinyl, $C_1$—$C_6$-alkylsulfonyl, hydroxy, $C_1$—$C_6$-alkoxy or trifluoromethyl;

Y is hydrogen, hydroxy, $C_1$—$C_6$-alkoxy,

$$-O\overset{O}{\overset{\|}{C}}NR^2R^3, \quad -O\overset{O}{\overset{\|}{C}}-R^9,$$

$$-NR^1_2, \quad -NH\overset{O}{\overset{\|}{C}}R^1 \quad \text{or}$$

$$-O\overset{O}{\overset{\|}{P}}(OH)OR^1;$$

W is hydrogen, hydroxy or $C_1$—$C_6$-alkoxy;

ring

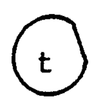

represents a fused thiophene or fused benzene ring said fused benzene ring optionally being substituted with a substituent 2 as defined below;

$R^2$ and $R^3$ are independently hydrogen (provided that both are not hydrogen, $C_1$—$C_6$-alkyl, phenyl-$C_1$—$C_6$-alkyl, cyclo-$C_2$—$C_7$-alkyl, phenyl or phenyl monosubstituted by $C_1$—$C_6$-alkyl, hydroxy, $C_1$—$C_6$-alkoxy, F, Cl, Br, I or trifluormethyl, hydroxy-$C_1$—$C_6$-alkyl, or $C_1$—$C_6$-alkoxy-$C_1$—$C_6$-alkyl;

in addition, when one of $R^2$ and $R^3$ is as defined above, the other may be —$R^4NR^5R^6$ { wherein$R^4$ is $C_1$—$C_6$-alkanediyl, $R^5$ is hydrogen or $C_1$—$C_6$-alkyl and $R^6$ is $C_1$—$C_6$-alkyl, or $R^5$ and $R^6$ together with the nitrogen atom form a 1-azetidinyl, 1-pyrrolidinyl, 1-piperidinyl, 1-(4-($C_1$—$C_6$)-alkylpiperazinyl), 4-morpholinyl or 1-hexahydroazepinyl group},

in further addition, $R^2$ and $R^3$ together with the nitrogen atom may form a 1-azetidinyl, 1-pyrrolidinyl, 1-piperidinyl, 4-morpholinyl, 1-(4-($C_1$—$C_6$)-alkylpiperazinyl), 1-(4-($C_1$—$C_6$)-alkoxy-($C_1$—$C_6$)-alkylpiperazinyl), 1-(4-hydroxy-($C_1$—$C_6$)-alkylpiperazinyl), 1-(3-hydroxyazetidinyl), 1-(3-($C_1$—$C_6$)-alkoxyazetidinyl), 1-(3-hydroxy-pyrrolidinyl), 1-(3-($C_1$—$C_6$)-alkoxypyrrolidinyl), 1-(3- or 4-hydroxypiperidinyl), 1-(3- or 4-($C_1$—$C_6$)-alkoxy-piperidinyl), 1-(4-oxopiperidinyl) or 1-(3-oxopyrrolidinyl) ring;

in still further addition, when $R^2$ is hydrogen, $R^3$ may be —$CHR^7CO_2R^8$, wherein $R^7$ and $R^8$ are independently hydrogen, $C_1$—$C_6$-alkyl or phenyl-$C_1$—$C_6$-alkyl;

$R^9$ is $C_1$—$C_6$-alkyl, phenyl-$C_1$—$C_6$-alkyl, phenyl or phenyl monosubstituted by $C_1$—$C_6$-alkyl, hydroxy, $C_1$—$C_6$-alkoxy, F, Cl, Br, I or trifluormethyl, $C_1$—$C_6$-alkoxy-$C_1$—$C_6$-alkyl, phenyloxy-$C_1$—$C_6$-alkyl, phenyl-$C_1$—$C_6$-alkoxy-$C_1$—$C_6$-alkyl, cyclo-$C_3$—$C_7$-alkyl-$C_1$—$C_6$-alkyl, $C_1$—$C_6$-alkoxycarbonyl-$C_1$—$C_6$-alkyl, cyclo-$C_3$—$C_7$-alkyl, 1-adamantyl, cyclo-$C_3$—$C_7$-alkoxy-$C_1$—$C_6$-alkyl, $C_1$—$C_6$-alkoxy, phenyl-$C_1$—$C_6$-alkoxy, cyclo-$C_1$—$C_7$-alkoxy, phenoxy or phenoxy monosubstituted by $C_1$—$C_6$-alkyl, hydroxy, $C_1$—$C_6$-alkoxy, F, Cl, Br, I or trifluormethyl or —$CHR^7NHR^8$ {wherein $R^7$ and $R^8$ are as defined above}; and

Z is X as defined above, amino, $C_1$—$C_6$-alkylamino or

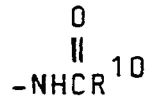

{wherein $R^{10}$ is hydrogen, $C_1$—$C_6$-alkyl or phenoxy or phenoxy monosubstituted by $C_1$—$C_6$-alkyl, hydroxy, $C_1$—$C_6$-alkoxy, F, Cl, Br, I or trifluormethyl.

Another aspect of the invention is a process for producing a compound having the structural formula I

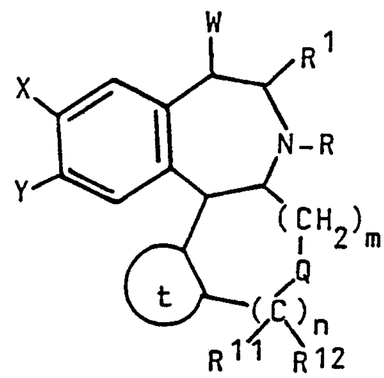

wherein:

R is hydrogen, $C_1$—$C_6$-alkyl, —$CH_2CH{=}CH_2$ or

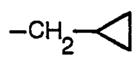 ;

$R^1$, $R^{11}$ and $R^{12}$ are the same or different and each is hydrogen or $C_1$—$C_6$-alkyl;

Q is methylene, —O— or —S—;

m and n are independently variable and may each have a value of 0, 1 or 2, with the provisos that the sum of m and n is not greater than 3, that m may not equal zero when Q is —O— or —S—, and that when Q is —$CH_2$— m and n cannot both be zero, unless W is H, X is $OCH_3$, Y is OH, B is H, $R^1$ is H and R is $CH_3$;

X is hydrogen, F, Cl, Br, I, $C_1$—$C_6$-alkyl, $C_1$—$C_6$-alkylthio, $C_1$—$C_6$-alkylsulfinyl, $C_1$—$C_6$-alkylsulfonyl, hydroxy, $C_1$—$C_6$-alkoxy or trifluoromethyl;

Y is hydrogen, hydroxy, $C_1$—$C_6$-alkoxy,

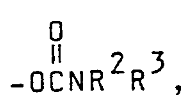 , 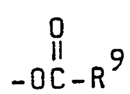 ,

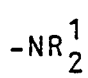 , 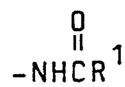 or

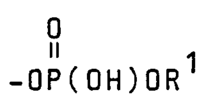 ;

W is hydrogen, hydroxy or $C_1$—$C_6$-alkoxy;

ring

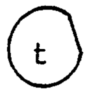

represents a fused thiophene or fused benzene ring said fused benzene ring optionally being substituted with a substituent 2 as defined below;

$R^2$ and $R^3$ are independently hydrogen (provided that both are not hydrogen, $C_1$—$C_6$-alkyl, phenyl-$C_1$—$C_6$-alkyl, cyclo-$C_3$—$C_7$-alkyl, phenoxy or phenoxy monosubstituted by $C_1$—$C_6$-alkyl, hydroxy, $C_1$—$C_6$-alkoxy, F, Cl, Br, I or trifluormethyl, hydroxy-$C_1$—$C_6$-alkyl, or $C_1$—$C_6$-alkoxy-$C_1$—$C_6$-alkyl;

in addition, when one of $R^2$ and $R^3$ is as defined above, the other may be —$R^4NR^5R^6$ {whereinR$^4$ is $C_1$—$C_6$-alkanediyl, $R^5$ is hydrogen or $C_1$—$C_6$-alkyl and $R^6$ is $C_1$—$C_6$-alkyl, or $R^5$ and $R^6$ together with the nitrogen atom form a 1-azetidinyl, 1-pyrrolidinyl, 1-piperidinyl, 1-(4-$C_1$—$C_6$-alkylpiperazinyl), 4-morpholinyl or 1-hexahydroazepinyl group},

in further addition, $R^2$ and $R^3$ together with the nitrogen atom may form a 1-azetidinyl, 1-pyrrolidinyl, 1-piperidinyl, 4-morpholinyl, 1-(4-($C_1$—$C_6$)-alkylpiperazinyl), 1-(4-($C_1$—$C_6$)-alkoxy-($C_1$—$C_6$)-alkylpiperazinyl), 1-(4-hydroxy-($C_1$—$C_6$)-alkylpiperazinyl), 1-(3-hydroxyazetidinyl), 1-(3-($C_1$—$C_6$)-alkoxyazetidinyl), 1-(3-hydroxy-pyrrolidinyl), 1-(3-($C_1$—$C_6$)-alkoxypyrrolidinyl), 1-(3- or 4-hydroxypiperidinyl), 1-(3- or 4-($C_1$—$C_6$)-alkoxy-piperidinyl), 1-(4-oxopiperidinyl) or 1-(3-oxopyrrolidinyl) ring;

in still further addition, when $R^2$ is hydrogen, $R^3$ may be —$CHR^7CO_2R^8$, wherein $R^7$ and $R^8$ are independently hydrogen, $C_1$—$C_6$-alkyl or phenyl-$C_1$—$C_6$-alkyl;

$R^9$ is $C_1$—$C_6$-alkyl, phenyl-$C_1$—$C_6$-alkyl, phenyl or phenyl monosubstituted by $C_1$—$C_6$-alkyl, hydroxy, $C_1$—$C_6$-alkoxy, F, Cl, Br, I or trifluormethyl, $C_1$—$C_6$-alkoxy-$C_1$—$C_6$-alkyl, phenyloxy-$C_1$—$C_6$-alkyl, phenyl-$C_1$—$C_6$-alkoxy-$C_1$—$C_6$-alkyl, cyclo-$C_3$—$C_7$-alkyl-$C_1$—$C_6$-alkyl, $C_1$—$C_6$-alkoxycarbonyl-$C_1$—$C_6$-alkyl, cyclo-$C_3$—$C_7$-alkyl, 1-adamantyl, cyclo-$C_3$—$C_7$-alkoxy-$C_1$—$C_6$-alkyl, $C_1$—$C_6$-alkoxy, phenyl-$C_1$—$C_6$-alkoxy, cyclo-$C_3$—$C_7$-alkoxy, phenoxy or phenoxy monosubstituted by $C_1$—$C_6$-alkyl, hydroxy, $C_1$—$C_6$-alkoxy, F, Cl, Br, I or trifluormethyl or —$CHR^7NHR^8$ {wherein $R^7$ and $R^8$ are as defined above}; and

Z is X as defined above, amino, $C_1$—$C_6$-alkylamino or

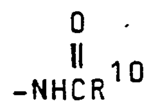

{wherein $R^{10}$ is hydrogen, $C_1$—$C_6$-alkyl or phenyl or phenyl monosubstituted by $C_1$—$C_6$-alkyl, hydroxy, $C_1$—$C_6$-alkoxy, F, Cl, Br, I or trifluormethyl} characterized by:

(A) intramolecular condensation of a compound of the formula

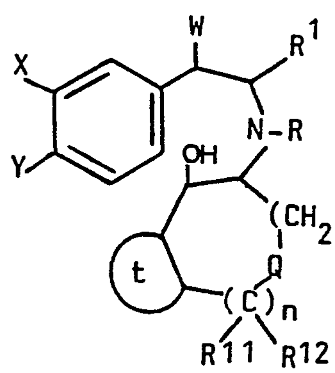

II

Compounds according to formula I may be prepared by intramolecular condensation of a compound having the structural formula II in the presence of a dehydration catalyst. Effective dehydration catalysts include sulfonic acid, methanesulfonic acid, trifluoromethanesulfonic acid, phosphoric acid and anhydrous hydrofluoric acid.

The intramolecular condensation described above may be performed at various temperatures and pressures, e.g., between 0°C and 100°C and at reduced, atmospheric or elevated pressure. An inert solvent may be employed or the reaction may be run in the absence of solvent. The time required for obtaining the desired product varies somewhat with the temperature, pressure and batch size but the reaction is generally complete within 24 hrs.

i. Compounds of formula II may be obtained by reacting an amine of formula V with a compound of formula VI

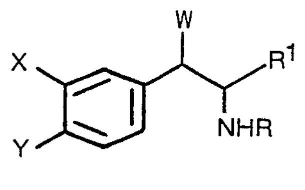

V

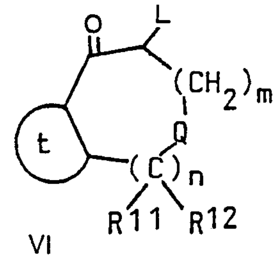

VI

to produce a compound of formula VII

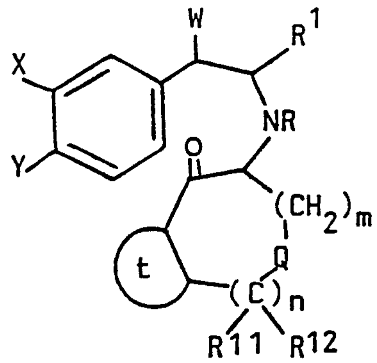

VII

followed by reduction of the carbonyl group in the product of formula VII to hydroxy. The symbol L represents a readily displaceable moiety commonly known as a "leaving group". Suitable leaving groups used by those skilled in the art include but are not limited to the halides, e.g., chlorine, bromine or iodine, and $OSO_2R$ wherein R may be hydrogen, $C_1$—$C_6$-alkyl, phenyl-$C_1$—$C_6$-alkyl, or phenyl or phenyl mono substituted by $C_1$—$C_6$-alkyl, hydroxy, $C_1$—$C_6$-alkoxy, F, Cl, Br, I or trifluormethyl (for example *para*-toluenesulfonyl). Preferred reducing agents for the reduction step include $NaBH_4$, $LiAlH_4$, $BH_3$, and $NaAlH_2(OCH_2CH_3OCH_3)_2$. Catalytic reductions using catalysts such as palladium on carbon or Raney nickel and hydrogen gas at 1 to 10 atmospheres pressure are also effective.

ii. Compounds of formula II may also be obtained by reacting an amine or formula VIII with a compound of formula IX under the conditions described in the preceding paragraph but without the reduction step:

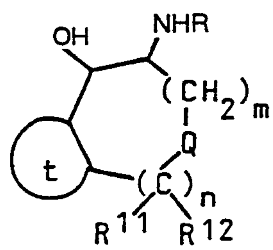

VIII

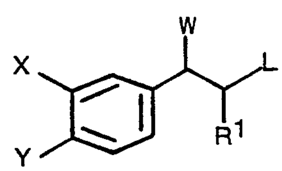

IX

iii. Compounds of formula II can be prepared by reacting an aldehyde ($R^1$ = H) or ketone ($R^1$ = $C_1$—$C_6$-alkyl) of formula X with an amino alcohol of formula VIII in the presence of an appropriate reducing agent such as sodium cyanoborohydride at pH 4—7.

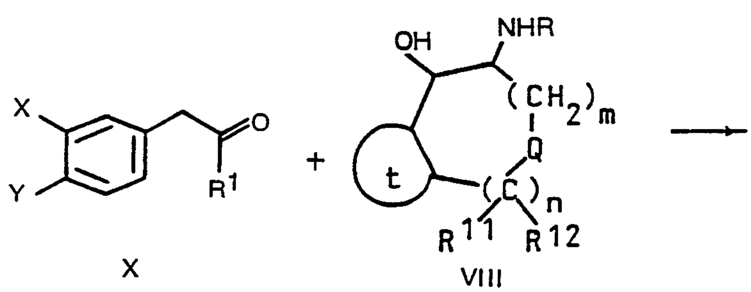

II

X VIII

iv. Compounds of formula II may also be prepared by reaction of compounds of formula VIII with aldehydes or ketones of formula X with prior removal of water of condensation followed by reduction of the resulting intermediate condensation product with reducing agents such as $NaBH_4$ or $NaCNBH_3$, or by catalytic reduction using catalysts such as palladium on carbon or Raney nickel under a hydrogen atmosphere at 1—10 atmospheres pressure.

(B) Compounds of formula I also may be prepared by reacting a compound of the formula

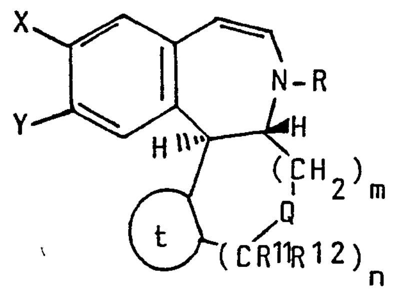

XI

with a suitable reducing agent to produce the compound of formula I. Preferred reducing agents comprise hydrogen and a catalyst with $PtO_2$ being a particularly preferred catalyst.

The compound of formula XI may be prepared, for example, by reasting a compound of formula XII with a 1-halo-2,2-di-($C_1$—$C_6$)-alkoxyethane (halo represents F, Cl, Br, I) in the presence of a suitable solvent such as dimethylformamide and a catalyst such as an alkali metal iodide, preferably potassium iodide, to produce a compound of formula XIII.

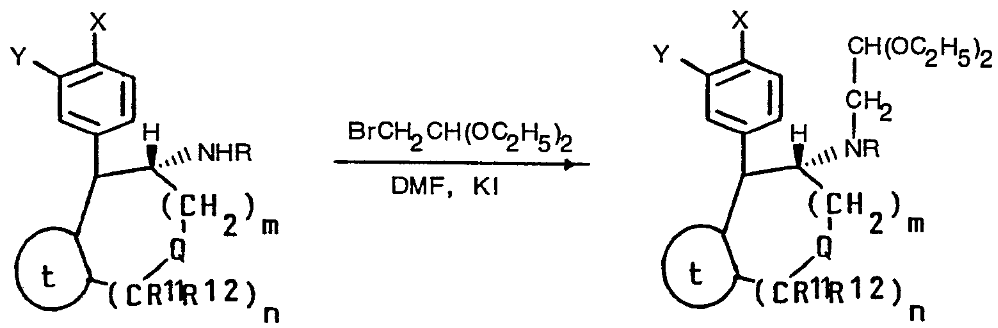


The compound of formula XIII may be reacted with a strong acid such as trifluoromethane sulfonic acid or sulfuric acid at a temperature ranging between about 0—25°C, to produce the compound of formula XI.

C. Compounds of formula I also may be prepared by reacting a compound of formula XIV

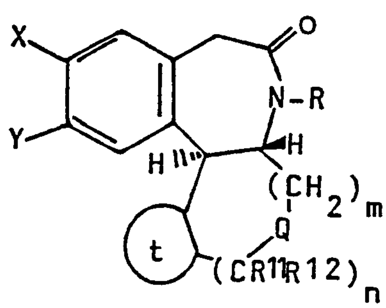

with a reducing agent such as $LiAlH_4$ or $BH_3$, preferably $BH_3$, at a temperature ranging between about 0°C and about 70°C to produce the compound of formula I.

One method for preparing the compund of formula XIV is set forth below. This method is particularly applicable where Y is OH or $C_1$—$C_6$-alkoxy. Where Y is one of the other substituents defined, additional process steps known in the art may be necessary in addition to those described below. Compound XIV may be prepared by first reacting a compound of formula XV with a compound of XVa

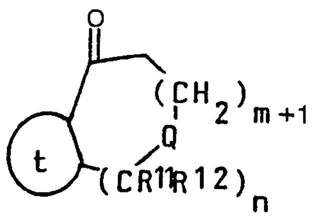

+

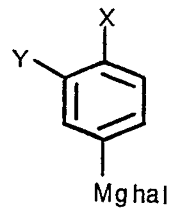

XV

XVa

to produce a compound of the formula XVI

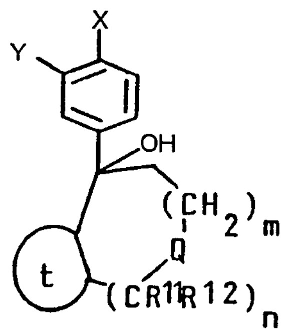

XVI

which may be dehydrated to a compound of formula XVII using acidic catalysts such as toluene sulfonic acid or phosphorus oxychloride/pyridine at a temperature ranging between about 20°C and about 120°C with continuous removal of water.

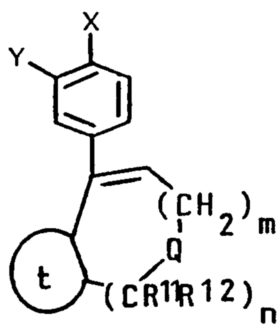

XVII

The compound of formula XVII may be oxidized to produce a compound of formula XVIII below using m-chloroperbenzoic acid at a temperature ranging between about 0°C and about 20°C, followed by contact with an alkali metal hydroxide such as sodium hydroxide, followed by contact with a strong mineral acid.

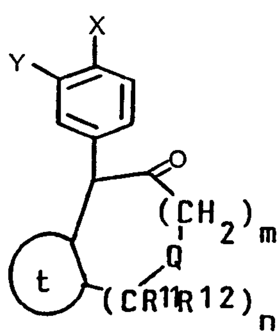

XVIII

The compund of formula XVIII may in turn be reacted with an $C_1$—$C_6$-alkyl amine with continuous removal of water to produce a compound of formula XIX

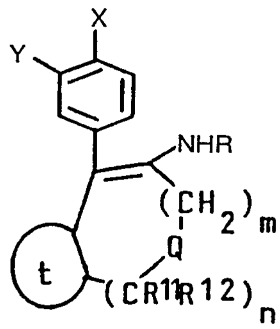

XIX

which then may be reduced in the presence of a catalyst such as zinc dust and acetic acid to produce a compound of formula XX

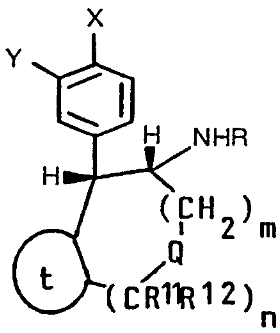

XX

which may then be treated with a strong base in dimethylsulfoxide (DMSO) or a mixed solvent comprising DMSO plus a polar aprotic solvent, e.g., dimethylformamide (DMF) to produce a compound of formula XXI. Strong bases utilized are preferably potassium tert butoxide or sodium hydride.

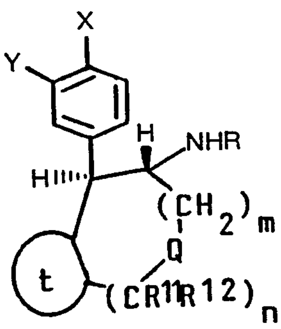

XXI

The compound of formula XXI may be contacted with a haloacetyl halide to produce a compound of formula XXII

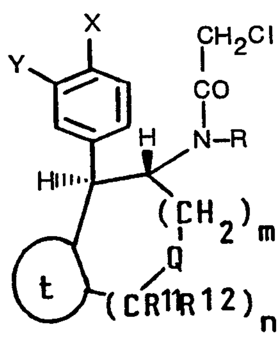

XXII

which may be exposed to light to produce the compound of formula XIV

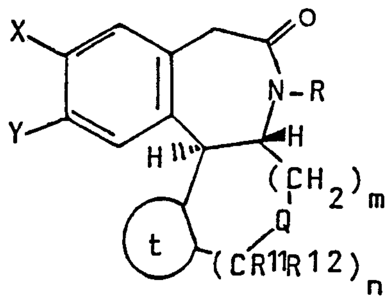

XIV

which may then be reduced to the compound of formula I.

In the above processes A—C, it is sometimes desirable and/or necessary to protect certain R, $R^1$, $R^{11}$, $R^{12}$, W, X, Y and Z groups during the reactions. Conventional protecting groups are operable. For example, the groups listed in column 1 of the following table may be protected as indicated in column 2 of the table:

| 1. Group to be Protected | 2. Protected Group |
|---|---|
| —COOH | —COO-$C_1$—$C_6$-alkyl, —COObenzyl, —COOphenyl |
| NH | N—$CO_2$-$C_1$—$C_6$-alkyl, N—$CO_2$benzyl, N—$CO_2CCH_2Cl_3$ |
| CO | 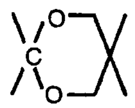 , 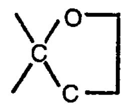 |
| —OH | 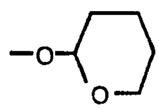 , $CH_3O$ |
| —$NH_2$ | 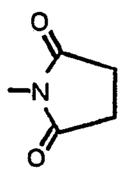 |

Of course, other protecting groups well known in the art may be used. After the reaction or reactions, the protecting groups may be removed by standard procedures.

Also, R, $R^1$, $R^{11}$, $R^{12}$, W, X, Y and Z groups in formula I may be varied by appropriate selection of starting materials from which the compounds are synthesized or by reacting a compound of formula I with a suitable reagent to effect the desired conversion of the substituent to another R, $R^1$, $R^{11}$, $R^{12}$, W, X, Y and Z group. The latter procedure is particularly applicable for changing the substituents X. For example, a chlorine substituent may be added in place of hydrogen by reaction with a chlorinating agent such as sulfuryl chloride in a non-reactive solvent. A hydroxymethyl substituent in the X position may be added in place of hydrogen by reaction with formaldehyde in a suitable solvent system, e.g., in a mixed solvent system consisting of dimethyoxyethane and aqueous potassium hydroxide, preferably at an elevated temperature. Such a hydroxymethyl substituent may be reduced to a methyl group by reaction with a catalyst such as palladium hydroxide in a hydrogen atmosphere under pressure. Methoxy substituents may be converted to hydroxy, e.g., by refluxing in a mixture of sodium hydride, DMF and ethanethiol, or by reaction with concentrated hydrobromic acid. Other substitutions may be accomplished using standard techniques.

When utilized herein, the following terms, unless otherwise specified, have the following scope:

halo — represents fluoro, chloro, bromo or iodo;

alkyl (including, for example, the alkyl portions of alkylthio, alkoxy, aralkyl, alkoxyalkoxy, etc.) — represents straight or branched carbon chains having 1 to 6 carbon atoms;

cycloalkyl groups (including the cycloalkyl portion in cycloalkoxy groups) — represents saturated carbocyclic rings having 3 to 7 carbon atoms;

alkanediyl — represents a divalent, straight or branched hydrocarbon chain having from 1 to 6 carbon atoms, the two available bonds being from the same or different carbon atoms thereof, e.g., methylene, ethylene, ethylidene, $-CH_2CH_2CH_2-$,

$$-CH_2CHCH_3,$$

$=CHCH_2CH_3$, etc.;

aryl (including, for example, the aryl moiety in aralkyl or aralkoxy groups) — represents unsubstituted phenyl and phenyl mono substituted by alkyl, hydroxy, alkoxy, halo or trifluoromethyl.

The compounds of formula I possess analgesic, anticholinergic, antiaggressive and general tranquilizing properties. The invention therefore includes the use of compounds of formula I in combination with a pharmaceutically acceptable carrier for the production of a medicament for treating mental disorders including psychoses, schizophrenia or depression in a mammal, or for the control of pain or anxiety in a mammal. The compounds of formula I provide a long duration of activity.

Certain compounds of formula I wherein X and Y are hydroxy and R is hydrogen are also active as renal vasodilators. These compounds can thus be used in pharmaceutical compositions in combination with a pharmaceutically acceptable carrier and in methods for controlling hypertension by administering to a mammal a renal vasodilating effective amount of such a compound.

Compounds according to formula II may exist as diastereomers. Specifically, the hydrogen bonded to the carbon bearing the hydroxyl group and the hydrogen bonded to the adjacent saturated carbon atom may be *cis* or *trans* to each other. Upon condensation, the fused ring systems of formula I may be joined *cis* (formula III) or *trans* (formula IV) and are, therefore, also diastereomers:

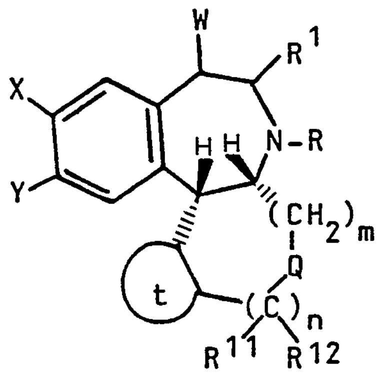

III

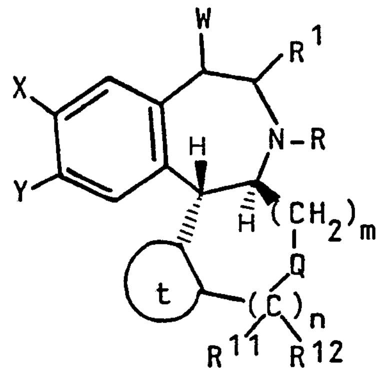

IV

The *trans* form (formula IV) of the compounds of formula I i.e. the compunds of formula IV is a preferred embodiment. It is noted that, when $R^1$ and/or W is other than hydrogen and when $R^{11}$ and $R^{12}$ are different at least one other assymetric center exists in the compounds of the invention. All such isomeric forms and mixtures thereof are within the scope of the present invention. Unless otherwise indicated, the methods of preparation disclosed herein result in product distributions which include all possible structural isomers, although it is understood that physiological response may vary according to stereochemical structure. The isomers may be separated by conventional means such as fractional crystallization or HPLC.

Ring

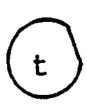

may represent a fused thiophene ring. The sulfur atom in such fused thiophene ring may be in any of the non-fused positions of said ring.

Compounds of formulas I and II can exist in unsolvated as well as solvated forms, including hydrated forms. In general, the solvated forms, with pharmaceutically acceptable solvents such as water, ethanol and the like, are equivalent to the unsolvated forms for purposes of this invention.

The compounds of formulas I and II may form pharmaceutically acceptable salts with organic and inorganic acids. Examples of suitable acids for salt formation are hydrochloric, sulfuric, phosphoric, acetic, citric, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous sodium hydroxide, potassium carbonate, ammonia and sodium bicarbonate. The free base forms differ

from their respective salt forms somewhat in certain physical properties, such as solubility in polar solvents, but the salts are otherwise equivalent to their respective free base forms for purposes of the invention.

The compounds of formula I display pharmacological activity in test procedures designed to indicate anti-psychotic and anti-depressive activity. The compounds are non-toxic at pharmaceutically therapeutic doses.

CONDITIONED AVOIDANCE SUPPRESSION IN RATS

Clinically active antipsychotic drugs are known to depress discrete trial avoidance behavior at doses that do not retard escape response (Ann. N. Y. Acad. Sci. *66*, 740 (1957)). A series of experiments was carried out to assess the ability of the compouunds of this invention to suppress the conditioned avoidance response (CAR) in rats.

Materials and Methods

Rats were required to jump onto a platorm located 6.75 inches (17.15 cm) above the grid floor of an experimental chamber in response to a 5-second tone to avoid a 10-second foot shock (0.6 ma). Each experimental session consisted of 20 such trials presented at 30-second intervals. A correct CAR is scored whenever the rat jumps onto the platform during the tone (prior to foot shock). An escape response is scored when the rat jumps onto the platform during a shock. A response failure is defined as the lack of an escape response during the 10-second shock period.

Groups 6—8 rats were trained in two consecutive days (total of 40 trials). Rats that reached criterion on day 2 (correct CARs on 16 or more of the 20 trials) were treated with either a test drug or vehicle on day 3. Suppression of CAR was analyzed statistically using Student's t-test comparing the performances of drug-treated to vehicle-treated rats. The minimal effective dose (MED) for each drug is defined as the lowest dose tested that significantly ($P < 0.05$) reduced avoidance responding.

Results

The results in the above procedure with representative compounds of the invention are shown in Column 8 of Table I below.

SQUIRREL MONKEY CONDITIONED AVOIDANCE RESPONSE (CAR) TEST

This test was designed to measure the effective duration of candidate compounds.

Male or female squirrel monkeys weighing 800—1200 g housed one per cage were utilized. Initially each monkey was taught to terminate a 3mA electric shock delivered through the grid floor of the test cage and an overlapping tone by depressing a lever in the cage. The monkeys did not proceed to the second phase of testing unless they depressed the lever during the shock component of the trials at least 75% of the time during 60 daily trials on three consecutive days.

In the second phase of the testing, a ten second tone is turned on prior to the shock component. A lever press during the sounding of the tone terminates the tone and prevents the occurrence of the shock component and is denoted as an "avoidance". Compound testing does not begin until the monkey makes at least 85% correct avoidances for five consecutive days.

The compound testing was commenced after three consecutive days of re-testing. The monkey first was injected or orally dosed with the vehicle only and retested to show that the vehicle does not affect the response of the monkey. The monkey must achieve at least an 85% correct avoidance before drug testing commences. If this minimal avoidance level is achieved, the next day the monkey is orally dosed or injected with the subject compound in the appropriate vehicle and the number of avoidances are recorded. An animal arbitrarily is defined as having been "affected" by any drug treatment if there is a 50% loss of avoidance behavior relative to the performance of the animal when only the vehicle was injected. The minimal effective dose (MED) is defined as that dose producing an effect in at least 50% of the animals.

A test was conducted to determine the effective duration of a compound of the present invention, 6,7,7a,8,9,13b-hexahydro-3-chloro-2-hydroxy-7-methyl-5*H*-benzo[d]naphtho[2,1-b]azepine, denoted as Compound A, compared to a known compound, (d)-7-chloro-8-hydroxy-3-methyl-1-phenyl-2,3,4,5-tetrahydro-1h-3-benzazepine maleate, denoted as Compound B (Sch 23390).

It was determined that the $ED_{50}$ (approximately 1.6 mg/kg po), of Compound A administered 60 minutes prior to the test, was roughly equivalent to the $ED_{50}$ (2.4 mg/kg po) of compound B administered 30 minutes prior to test. The duration of each compound was determined by administering a 10 mg/kg po dose six hours prior to testing. The ability to significantly decrease the number of avoidances six hours after injection was used to indicate that the compound was still active at that time. The tests showed that Compound A caused a significant decrease in the number of avoidances ($p < 0.05$) whereas Compound B was inactive at that time. These results are presented below in Table II where N is the number of monkeys tested.

TABLE II

| Treatment | Dose | N (Mg/Kg) | Mean (±SE) Number of Avoidances at 6 hours Post-Treatment |
|---|---|---|---|
| Vehicle | — | 7 | 59.1 + 0.9 |
| Compound A | 10 | 7 | 23.9 + 7.5 |
| Compound B | 10 | 3 | 59.7 + 0.3 |

COMPETITIVE INHIBITION ASSAY

Many compounds capable of effecting reproducible physiological changes in neural tissues are believed to operate by binding at one or more receptor sites. Compounds which interact strongly with these receptor sites in *in vitro* tests, using homogenates of the target organ or structure, are expected to exhibit similar properties when administered *in vivo* and are, therefore, candidates for continued study as potential therapeutic and/or diagnostic agents.

Binding of a compound to a receptor site, *in vitro*, is demonstrated by the specificity of binding and the saturability of the available sites. A methodology for characterization of D—1 and D—2 receptor binding and an interpretation of the data are described by Billard et al., *Life Sciences 35*, 1885 (1984) in which the binding of the benzazepine (R)-(+)-8-chloro-2,3,4,5-tetrahydro-3-methyl-5-phenyl-1*H*-3-benzazepin-7-ol hemimaleate (SCH 23390) to the dopamine D—1 receptor is characterized. A selectivity for D—1 receptor binding as compared to D—2 receptor binding is believed to confer the therapeutic advantage of avoiding troublesome and potentially irreversible neurological side effects associated with D—2 receptor occupancy.

Materials and Methods

Tritiated SCH 23390 and tritiated spiperone (a potent D—2 receptor ligand) were obtained as described in the Billard et al. reference *supra* and serially diluted in 0.05 M Tris buffer, pH 7.4, as required. Compounds of this invention were synthesized as disclosed herein and diluted in 0.05 M Tris buffer, pH 7.4, as required.

Tissue Preparation

Male Sprague-Dawley rats (200 to 250 g) from Charles River Breeding Laboratories, Mass. were used to obtain brain tissue. The rats were humanely sacrificed and their brains removed and placed on ice. Striatal tissue was excises, pooled, and homogenized (Brinkman Polytron, 10 sec) in 100 volumes (w/v) of ice cold 50 mM Tris buffer, pH 7.4 (at 25°C). The homogenate was centrifuged at 20,000 xg for 10 min. The resultant pellet was rehomogenized in Tris buffer and centrifuged again. The final pellet was resuspended in 50 mM Tris buffer pH 7.4 containing 120 mM NaCl, 5 mM KCl, 2 mM $CaCl_2$, and 1 mM $MgCl_2$.

Assay

Polypropylene incubation tubes received 100 µl of the individual test compounds at various concentrations dissolved or suspended in 0.05 M Tris, pH 7.4 containing 4 mg/ml methylcellulose, 100 µl of a solution of $^3H$—SCH 23390 in Tris buffer (final reaction mixture concentration =0.3 nM) or 100 µl of a solution of $^3H$-spiperone in Tris buffer (final concentration = 0.2 nM) and 800 µl of tissue suspension (ca. 3 mg/assay). Tubes were incubated at 37°C for 15 minutes and rapidly vacuum filtered through Whatman® GF/B filters and rinsed 4 times with 4 ml of ice cold 50 mM Tris buffer, pH 7.4. The filters were transferred to scintillation vials, equilibrated with 10 ml of scintillant (Scintosol, Isolab, Inc.) for 16 hours at 25°C and the radioactivity determined in liquid scintillation counter. $K_i$ values were determined as described by Billard et al. using the relationship $K_{5I} = IC_{50}/(1 + ([L]/K_D))$ wherein $IC_{50}$ = concentration of test drug necessary to displace 50% of specifically bouund $^3H$—Sch 23390, [L] = concentration of radioligand used in the assay and $K_D$ = dissociation constant.

Results

The inhibition constants ($K_i$) determined from the assays for a series of compounds of the invention are as shown in columns 6 and 7 of Table I below.

TABLE I

| Col. 1 | Col. 2 | Col. 3 | Col. 4 | Col. 5 | Col. 6 | Col. 7 | Col. 8 |
|---|---|---|---|---|---|---|---|
| | | stereo-chemistry of 7a and 7b | | | Ki (nM) | | |
| Q | n | H's | X | Y | $^3$H-23390 | $^3$H-Spip | CAR(MED) |
| $CH_2$ | 1 | cis | $CH_3O$ | OH | 6450 | >100,000 | |
| $CH_2$ | 1 | cis | HO | $CH_3O$ | 44,800 | >100,000 | |
| $CH_2$ | 1 | trans | $CH_3O$ | OH | 23 | 2500 | 30(po); 0.3-1(sc) |
| $CH_2$ | 1 | trans | HO | $CH_3O$ | 2970 | >100,000 | |
| $CH_2$ | 1 | trans | Cl | OH | 5.5 | 11,500 | 30(po);0.3(sc) |
| $CH_2$ | 1 | 7b(S):7a(R)(+) | Cl | OH | 1800 | >100,000 | >30 (po) |
| $CH_2$ | 1 | 7b(R):7a(S)(-) | Cl | OH | 12 | 14,300 | 30 (po) |
| $CH_2$ | 1 | cis | Cl | OH | 6200 | >100,000 | |
| $CH_2$ | 1 | trans | H | OH | 80 | 3500 | |
| $CH_2$ | 2 | trans | $CH_3O$ | OH | 292 | >100,000 | 10 (sc) |
| $CH_2$ | 2 | trans | HO | $CH_3O$ | 7730 | >100,000 | 10 (sc) |
| $CH_2$ | 1 | trans | $CH_3$ | OH | 119 | 7200 | |
| $CH_2$ | 1 | trans | Cl | $NH_2$ | 70 | 4175 | 3 (po) |
| O | 1 | trans | H | OH | 121 | - | |

The comparatively small $K_i$ values of these compounds in the competitive binding assay with SCH 23390 indicate that the compounds of formula I bind strongly to the D—1 receptor site. The relatively high $K_i$ values for the D—2 site, for which spiperone is highly selective, indicate that the compounds are not specifically bound to that receptor site.

The antidepressive method of the invention is identified, for example, by test procedures which measure a compound's effect on tetrabenazine (TBZ)-induced ptosis in mice or which measure a compound's effect on muricide activity in rats as discussed below.

ANTIDEPRESSANT POTENTIAL

EFFECTS ON TETRABENAZINE (TBZ)-INDUCED PTOSIS IN MICE

Clinically active antidepressant drugs are known to block TBZ-induced ptosis in mice (Psychosomatic Medicine, Nodine and Moyer, Eds., Lea and Febiger, Philadelphia, 1962, pp 683—90). Activity in this test is used to predict anti-depressant activity in man.

Methods and Materials

Groups of 5 mice are administered test drugs followed 30 minutes later by ip injection of tetrabenazine, 30 mg/kg. Thirty minutes later, the degree of ptosis is evaluated. Percenbt blockade of each treated group is used to determine $ED_{50}$'s, defined as that dose which prevents ptosis in 50% of mice. $ED_{50}$'s and 95% confidence limits are calculated by probit analysis.

EFFECTS ON MURICIDAL BEHAVIOR IN RATS

Blockade of muricidal (mouse-killing) behavior in rats is used as a measure of evaluating the antidepressant activity of drugs (Int. J. Neuro-pharmacol., *5*, 405—11 (1966)).

Methods and Materials

Groups of 5 rats are administered test drug intraperitonially and are tested 30 and 60 minutes later for presence of muricidal behavior. Percent blockade of each treated group using data obtained at both these time points is calculated and dose-response data are used to determine each $ED_{50}$. $ED_{50}$ is defined as that dose which blocks muricide behavior in 50% of treated rats and is calculated using probit analysis.

The analgesic effect of the compounds of formula I and the method for providing analgesia may be exemplified by the Acetic Acid Writhing Test in Mice described below.

ACETIC ACID WRITHING TEST IN MICE

The blockade of writhing induced by the intraperitoneal injection of acetic acid is an established experimental animal model for the screening of antinociceptive drugs (drugs which prevent the appreciation or transmission of pain sensations). See Hendershot et al., *J. Pharmacol. Exp. Therap. 125*: 237, (1959) and Koster et al., *Fed. Proc. 18*: 412, (1959).

Methods and Materials

Compounds to be tested are dissolved or suspended in aqueous 0.4% methylcellulose vehicle. For oral administration, dosages are prepared for delivery of the selected weight of compound in a total volume of 20 mg/kg of body weight. For subcutaneous or intraperitoneal administration, dosages are prepared for delivery of the selected weight of compound in a volume of 10 ml/kg of body weight.

The test procedure is that described by Hendershot et al., *supra*, except that acetic acid is substituted for phenylquinone. Groups of five male CF1 mice (20—26 g) are dosed orally with test drug and injected 15 minutes later with 0.6% aqueous acetic acid (10 mg/kg). The mice are placed in a large observation beaker and the number of writhes for each animal is counted during a 10 minute interval starting 3 minutes after injection of acetic acid. A writhe is defined as a sequence of arching of the back, pelvic rotation and hindlimb extension. Initial screening is performed using a dosage of 30 mg/kg. If this dose affords 50% or greater reduction in the number of writhes compared to the control, the animal is considered to be protected, a dose response curve is developed using a logarithmic sequence of lower doses and an $ED_{50}$ is determined by interpolation.

The renal vasodilation effect of compounds of formula I wherein X and Y are both hydroxy and R is hydrogen (i.e., the other substituents, e.g., $R^1$, Q, m and n can be varied as described above) can be demonstrated by test procedures which measure renal arterial blood flow, such as that described by McNay et al., *J. Pharmacol. and Exptl. Therap. 151*, 23 (1966), or which measure renal vascular resistance, such as that described by Weinstock et al., *J. Med. Chem. 23*, 973 (1980).

For preparing pharmaceutical compositions from the compounds of formula I, inert, pharmaceutically acceptable carriers are admixed with the active compounds. The pharmaceutically acceptable carriers may be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents; it may also be an encapsulating material.

Liquid form preparations include solutions, suspensions and emulsions. As an example may be mentioned water or water-propylene glycol solutions for parenteral injection.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit.

The invention also contemplates alternative delivery systems including, but not necessarily limited to, transdermal delivery. The transdermal compositions can take the form of creams, lotions and/or emulsions and can be included in a transdermal patch of the matrix or reservoir type as are conventional in the art for this purpose.

Preferably, the pharmaceutical preparation is in unit dosage form. In such form, the preparation is subdivided into unit doses containing appropriate quantities of the active components. The unit dosage form can be a packaged preparation, the package containing discrete quantities of preparation such as packeted tablets, capsules and powders in vials or ampules. The unit dosage form can also be a capsule, cachet or tablet itself, or it may be the appropriate number of any of these in a packaged form.

The quantity of active compound in a unit dose preparation may be varied or adjusted from 1 mg to 100 mg according to the particular application and the potency of the active ingredient and the intended treatment. This would correspond to a dose of about 0.02 to about 2.0 mg/kg which may be divided over 1 to 3 administrations per day. The composition may, if desired, also contain other therapeutic agents.

The dosages may be varied depending on the requirement of the patent, the severity of the condition being treating and the particular compound being employed. Determination of the proper dosage for a particular situation is within the skill of those in the medical art. For convenience, the total daily dosage may be divided and administered in portions throughout the day or by means providing continuous delivery.

The invention disclosed herein is exemplified by the following preparative examples.

## Example 1

(a) cis/trans-N-Methyl-N-(β-3,4-dimethoxyphenylethyl)-2-amino-1,2,3,4-tetrahydro-1-naphthol

To a stirred solution of 94.0 g of N-methylhomoveratryl amine and 70 g of anhydrous potassium carbonate in 600 ml of dry dimethylformamide (DMF) was added in a period of 30 min a solution of 108 g of 2-bromo-α-tetralone in 100 ml of dry DMF. The mixture was stirred at room temperature for 3 hours and then diluted with 5 liters of ice water. The mixture was extracted with 2 × 800 ml of ether and the combined ether extracts were washed with 2 × 500 ml of water. The residue resulting from evaporation of the dried ether layer was dissolved in 800 ml of anhydrous ethanol, was treated with 14.0 g of sodium borohydride (with cooling) and stirred for an additional 18 hours. After removing the solvent, the residue, in 500 ml of water, was heated on a steam bath for 30 min. After cooling, the aqueous mixture was extracted with 1 liter of ether. The ether layer was extracted with 700 ml of 1N HCl, and the acid extract was made slightly basic with sodium hydroxide and extracted with 1 liter of ether. Upon evaporation of the ether, the residue was chromatographed over silica gel to give *cis/trans*-N-methyl-N-(β-3,4-dimethoxyphenylethyl)-2-amino-1,2,3,4-tetrahydro-1-naphthol as a viscous gum.

(b) cis/trans-6,7,7a,8,9,13b-Hexahydro-2,3-dimethoxy-7-methyl-5H-benzo[d]naptho[2,1-d]azepine

To 53.7 g of *cis/trans*-N-methyl-N-(β-3,4-dimethoxyphenylethyl)-2-amino-1,2,3,4-tetrahydro-1-naphthol was added 400 ml of anhydrous methanesulfonic acid with cooling and stirring. The mixture was stirred at room temperature for 4 additional hours, diluted with 2 liters of ice and water and made basic with 50% sodium hydroxide. The basic solution was chilled to 20°C and then extracted with 500 ml of methylene chloride, followed by extraction with 500 ml of ether. Chromatography of the residue obtained by evaporation of the combined organic extracts over silica gel (ethyl acetate:ethanol:ammonium hydroxide, 100:3:1) afforded the *cis* isoemr having a melting point of 114—116°C and the *trans* isomer as a colorless gum, the maleate derivative thereof having a melting point of 149—152°C.

## Example 2

trans-6,7,7a,8,9,13b-Hexahydro-2-hydroxy-3-methoxy-7-methyl-5H-benzo[d]naphtho[2,1-b]azepine and trans-6,7,7a,8,9,13b-Hexahydro-3-hydroxy-2-methoxy-7-methyl-5H-benzo[d]naphtho[2,1-b]azepine

To a stirred suspension of 6.1 g of 50% sodium hydride in 75 ml of dry DMF was added slowly a solution of 7.8 g of ethanethiol in 100 ml of dry DMF. After stirring for 20 min, a solution of 16.2 g of *trans*-6,7,7a,8,9,13b-hexahydro-2,3-dimethoxy-7-methyl-5*H*-benzo[d]naphtho[2,1-b]azepine in 75 ml of DMF was added over a period of 5 min. The mixture was heated slowly to reflux and maintained at reflux for 45 min. The heat source was removed and the reaction mixture was first chilled to 50°C and then poured into 1.5 Kg of ice and water with stirring. The pH of the solution was adjusted to 8 by dropwise addition of acetic acid and the precipitate was removed by filtration. Fractional crystallization with, first chloroform-ethanol and then with acetonitrile, afforded *trans*-6,7,7a,8,9,13b-hexahydro-3-hydroxy-2-methoxy-7-methyl-5*H*-benzo-[d]naphtho[2,1-b]azepine, m.p. 229—231°C and *trans*-6,7,7a,8,9,13b-hexahydro-2-hydroxy-3-methoxy-7-methyl-5*H*-benzo[d]naphtho[2,1-b]azepine, m.p. 194—196°C.

Example 3

(a) *trans*-6,7,7a,8,9,13b-Hexahydro-2-methoxy-7-methyl-3-[5-(1-phenyl-1H-tetrazolyl)oxy]-5H-benzo[d]-naphtho[2,1-b]azepine

To a suspension of 5.2 g of *trans*-6,7,7a,8,9,13b-hexahydro-3-hydroxy-2-methoxy-7-methyl-5*H*-benzo-[d]naphtho[2,1-b]azepine in 40 ml of dry DMF was added, in small portions, 850 mg of 50% sodium hydride and the mixture was stirred for an additional 20 min at room temperature. A solution of 3.18 g of 5-chloro-phenyl-1*H*-tetrazole in 10 ml of DMF was added dropwise. After stirring for 2 hours at room temperature, the mixture was poured into 350 ml of ice and water. The solid obtained was recrystallized from ether to give *trans*-6,7,7a,8,9,13b-hexahydro-2-methoxy-7-methyl-3-[5-(1-phenyl-1*H*-tetrazolyl)oxy])5*H*-benzo[d]-naphtho[2,1-b]-6-azepine, m.p. 190—192°C.

(b) trans-6,7,7a,8,9,13b-Hexahydro-2-methoxy-7-methyl-5H-benzo[d]naphtho[2,1-b]azepine

To a solution of 6.8 g of *trans*-6,7,7a,8,9,13b-hexahydro-2-methoxy-7-methyl-3-[5(1-phenyl-1*H*-tetrazolyl)oxy]-5*H*-benzo[d]naphtho[2,1-b]azepine in 100 ml of acetic acid was added 750 mg of 20% palladium hydroxide on carbon. The resulting mixture was then hydrogenated at 60 psi at 55°C for 5.5 hours. After removing both catalyst and solvent, the residue was treated with 150 ml of ether and 50 ml of 1N sodium hydroxide. The residue obtained from evaporation of the dried ether layer was recrystallized from ether to give *trans*-6,7,7a,8,9,13b-hexahydro-2-methoxy-7-methyl-5*H*-benzo[d]naphtho[2,1-b]azepine, m.p. 96—98°C.

Example 4

trans-6,7,7a,8,9,13b-Hexahydro-2-hydroxy-7-methyl-5H-benzo[d]naphtho[2,1-b]azepine

To 2.0 g of *trans*-6,7,7a,8,9,13b-hexahydro-2-methoxy-7-methyl-5*H*-benzo[d]naphtho[2,1-b]azepine was added 20 ml of 48% hydrobromic acid and the mixture was heated in an oil bath at 130°C, with stirring, for 4.5 hours. The excess hydrobromic acid was distilled away and the residue was dissolved in 200 ml of boiling water. The pH of the hot aqueous solution was adjusted to 8 with sodium bicarbonate and the solution was cooled to 10°C. The precipitated solid was recrystallized from acetonitrile to afford *trans*-6,7,7a,8,9,13b-hexahydro-2-hydroxy-7-methyl-5*H*-benzo[d]naphtho[2,1-b]azepine, m.p. 209—211°C.

Example 5

trans-6,7,7a,8,9,13b-Hexahydro-3-chloro-2-hydroxy-7-methyl-5H-benzo[d]naphtho[2,1-b]azepine

To a stirred suspension of 0.45 g each of *trans*-6,7,7a,8,9,13b-hexahydro-2-hydroxy-7-methyl-5*H*-benzo[d]naphtho[2,1-b]azepine and silica gel (60—200 mesh) in 30 ml of tetrahydrofuran was added 0.14 ml of sulfuryl chloride. The suspension was stirred for 20 min at room temperature and the solvent removed at 25°C by vacuum evaporation. The residue was treated with 10 ml of water and 30 ml of chloroform, and the pH was adjusted to 8 by addition of solid sodium bicarbonate. The organic layer was dried, filtered and evaporated to yield a gummy residue which was chromatographed on silica gel (chloroform:ethanol:ammonium hydroxide — 100:4:1.5) to yield *trans*-6,7,7a,8,9,13b-hexahydro-3-chloro-2-hydroxy-7-methyl-5*H*-benzo[d]naphtho[2,1-b]azepine, m.p. 215—216°C after recrystallization from acetonitrile.

Example 6

(a) trans-6,7,7a,8,9,13b-Hexahydro-2-hydroxy-3-hydroxymethyl-7-methyl-5H-benzo[d]naphtho[2,1-b]-azepine

To a solution of 515 mg of *trans*-6,7,7a,8,9,13b-hexahydro-2-hydroxy-7-methyl-5*H*-benzo[d]naphtho-[2,1-b]azepine in 7.0 ml each of dimethoxyethane and 3.3% of aqueous potassium hydroxide was added 0.6 ml of 37% formaldehyde and the reaction was stirred at 80°C for 3.5 hours. The solvents were removed by evaporation and the resulting residue was dissolved in 20 ml of water. The pH of the aqueous solution was first adjusted to 8 by dropwise addition of acetic acid and then was extracted twice with 25 ml portions of chloroform. The residue obtained by evaporation of the organic layer was purified by chromatography on a silica gel column (chloroform:ethanol:ammonium hydroxide — 50:3:1) to give *trans*-6,7,7a,8,9,13b-hexahydro-2-hydroxy-3-hydroxymethyl-7-methyl-5*H*-benzo[d]naphtho[2,1-b]azepine, as a colorless gum.

(b) trans-6,7,7a,8,9,13b-Hexahydro-2-hydroxy-3,7,dimethyl-5H-benzo[d]naphtho[2,1-b]azepine

To a solution of 500 mg of *trans*-6,7,7a,8,9,13b-hexahydro-2-hydroxy-3-hydroxymethyl-7-methyl-5*H*-benzo[d]naphtho[2,1-b]azepine and 0.6 g of *p*-toluenesulfonic acid monohydrate in 20 ml of glacial acetic acid was added 60 mg of 20% palladium hydroxide on carbon and the mixture was heated to 60°C for 18 hours under a hydrogen atmosphere at a pressure of 413.7 kPa (60 psi (ca 4.2 kg/cm$^2$)). After removal of catalyst and solvent, the residue was dissolved in 3 ml of DMF and then poured onto a stirred solution of 75 ml of DMF and then poured onto a stirred solution of 75 ml of 3% sodium bicarbonate. The solid was filtered and subsequently purified by chromatography on silica gel (chloroform:ethanol:ammonium hydroxide — 50:3:1) to afford *trans*-6,7,7a,8,9,13b-hexahydro-2-hydroxy-3-methyl-7-methyl-5*H*-benzo[d]-naphtho[2,1-b]azepine, m.p. 235—237°C.

The stereochemistry of the above-described compounds was determined by nuclear magnetic resonance and the assignments confirmed on a selected compound, *trans*-6,7,7a,8,9,13b-hexahydro-2-hydroxy-3-methoxy-7-methyl-5*H*-benzo[d]naphtho[2,1-b]azepine, by X-ray crystallography.

Example 7

(+)-trans-6,7,7a,8,9,13b-Hexahydro-3-chloro-2-methoxy-7-methyl-5H-benzo[d]naphtho[2,1-b]azepine

A mixture of 2.70 g of *trans*-6,7,7a,8,9,13b-hexahydro-2-methoxy-7-methyl-5*H*-benzo[d]naphtho[2,1-b]-azepine and 3.18 g of di-p-tolyl-*d*-tartaric acid in 35 ml of ethyl acetate was heated on a steambath. After initial dissolution, a solid formed. The mixture was cooled to room temperature and the precipitated solids filtered. The wet filtered solids were digested for 10 minutes with 50 ml ethanol on a steambath, the mixture cooled, and solids filtered. This material was dissolved in 75 ml of 95% ethanol with heating. The solution was poured into a beaker and allowed to evaporate slowly at room temperature to 50 ml. Solids were filtered and washed with cold ethanol to give 2.5 g product, m.p. 173—175°C. This was redissolved in 100 ml of 95% ethanol, and the resulting solution allowed to slowly partially evaporate. The solids formed were filtered to give 462 mg of the title compound as the tartarate salt, m.p. 188—189°C.

This material was dissolved in water and treated with aqueous NaOH to give the title compound as the free base, which is a syrup, $[\alpha]_D^{25}$ 209.1 (C 1.25, $C_2H_5OH$).

Example 8

(+)-trans-6,7,7a,8,9,13b-hexahydro-3-chloro-2-hydroxy-7-methyl-5H-benzo[d]naphtho[2,1-b]azepine

190 mg of (+)-trans-6,7,7a,8,9,13b-hexahydro-2-hydroxy-7-methyl-5H-benzo[d]naphtho[2,1-b]azepine was heated in a mixture of 2 ml each of 48% HBr and 2 ml of acetic acid for 5½ hrs. The mixture was evaporated almost to dryness at 120°C in vacuo and 5 ml of water added to the residue, which was then heated on a steambath for 5 minutes. The aqueous solution then poured into a solution of 2 g of $NaHCO_3$ in 40 ml of $H_2O$. The insoluble residue was taken up in hot dimethylformamide (DMF) and added to the $NaHCO_3$ solution as well. Precipitated material was filtered off, washed with water and air-dried.

Recrystallization from acetonitrile gave 130 mg of the title compound, m.p. 239—241°, $[\alpha]_D^{25}$ 220.5 (C 0.29, DMF).

Example 9

(−)-trans-6,7,7a,8,9,13b-Hexahydro-3-chloro-2-methoxy-7-methyl-5H-benzo[d]naphtho[2,1-b]azepine

By basically the same procedure as described in Example 7 above but using di-p-tolyl-*L*-tartaric acid, one obtains the title compound as the tartarate salt, m.p. 188—189°C, which gave the oily base, $[\alpha]_D^{25}$ −204.9 (C 1.17 $C_2H_5OH$), on treatment with aqueous NaOH.

Example 10

(−)-trans-6,7,7a,8,9,13b-Hexahydro-3-chloro-2-hydroxy-7-methyl-5H-benzo[d]naphtho[2,1-b]azepine

From (−)-*trans*-6,7,7a,8,9,13b-hexahydro-2-methoxy-7-methyl-5H-benzo[d]naphtho[2,1-b]azepine in a manner analogous to that described in Example 8 above, one obtains the title compound, m.p. 239—41°C, $[\alpha]_D^{25}$ −235/1° (C 0.295, DMF).

Example 11

(a) 1-(3-Methoxyphenyl)-3,4-dihydronaphthalene

A solution of the Grignard reagent derived from 50 g (.27 mol) of 3-bromoanisole in 200 ml of dry THF was treated with a solution of alpha-tetralone in 100 ml of THF added over 1 hour while maintaining the internal temperature at 15—25°C. After stirring overnight, the mixture was diluted with 500 ml of ether and quenched with 200 ml of 20% aqueous ammonium chloride. The aqueous layer was extracted with 200 ml of ether. The combined organic layers were dried over $MgSO_4$ and evaporated to 66.5 g of the oily alcohol. This was dissolved in 450 ml of dry toluene containing .020 g of p-toluenesulfonic acid and refluxed overnight in a Dean-Stark apparatus. After cooling, the mixture was washed with 100 ml of 5% sodium bicarbonate, with 100 ml of water, and then with 100 ml of brine. The mixture was dried over $MgSO_4$ and evaporated. The residue was distilled under reduced pressure to give the title compound, b.p. (66.66Pa (.5 mm)) 158—162°C.

(b) 1-(3-Methoxyphenyl)-2-oxo-1,2,3,4-tetrahydronaphthalene

To a 2-phase mixture of 25.1 g (.106 mol) of 1-(3-methoxyphenyl)-2-oxo-1,2,3,4-tetrahydronaphthalene, 20 g (.238 mol) of sodium bicarbonate, 200 ml of water and 400 ml of methylene chloride at 5°C was added 24.4 g of 80—85% m-chloroperbenzoic acid portion-wise over 15 minutes. The internal temperature rose to 10°C. After the addition was complete, the mixture was stirred at 0—5°C for 2 hr. The layers were separated and the aqueous layer was extracted with 100 ml of methylene chloride. The combined organic layers were extracted with 200 ml of 10% sodium carbonate and then with 100 ml of water, dried over $MgSO_4$ and evaporated to an oil. IR, NMR, and thin layer chromatography indicated the presence of a mixture of epoxide and 1,2-diol monoester. The crude mixture was stirred 3 hours at room temperature in 1 M solution of sodium hydroxide in 5:1 ethanol-water. After adjusting the pH to 8 with HCl, the bulk of the solvent was evaporated. The residue was taken up in 500 ml of ether, which was then washed with 50 ml of water, dried over $MgSO_4$, and evaporated. The oily residue was dissolved in 300 ml of dry toluene containing .050 g of p-toluenesulfonic acid and refluxed 3 hour in a Dean-Stark apparatus. The solution was washed with 50 ml of 5% sodium bicarbonate and with 50 ml of water, dried over $MgSO_4$ and evaporated to

give 25.1 g of the title compund which was characterized as its 2,4-dinitrophenyl hydrazone derivative, m.p. 161—161.5°C (ethanol).

(c) 1-(3-Methoxyphenyl)-2-(N-methylamino)-3,4-dihydronaphthalene

Dry methylamine gas was bubbled through a refluxing solution of 18.0 g of 1-(3-methoxyphenyl)-2-oxo-1,2,3,4-tetrahydronaphthalene in 300 ml of toluene in a Dean-Stark apparatus. After 1.5 hour the mixture was cooled and evaporated to give the title compound as a semi-solid enamine which was characterized by NMR δ 2.70 (S, 3H), 2.3—3.0 (M, 4H), 3.80 (S, 3H), 6.7—7.5 (M, 8H).

(d) cis-1-(3-Methoxyphenyl)-2-(N-methylamino)-1,2,3,4-tetrahydronaphthalene

Zinc dust (21 g, 321 mol) was activated by washing with 1M HCl, water, methanol, and ether, and then drying in vacuo. To the suspension of zinc dust in 300 ml of glacial acetic acid was added 17.0 g of 1-(3-methoxyphenyl)-2-(N-methylamino)-3,4-dihydronaphthalene, and the mixture was stirred at 100°C for 16 hrs. The mixture was cooled, filtered, and evaporated to dryness. The residue was dissolved in 100 ml of .5M HCl and extracted with 100 ml of ether. The aqueous layer was made strongly basic with solid KOH, then extracted twice with 300 ml of ethyl acetate. The ethyl acetate was dried over $MgSO_4$ and evaporated to an oil which was transformed into its HCl salt by ethereal HCl to provide the title compound as its hydrochloride salt, m.p. 172—174°C.

(e) trans-1-(3-Methoxyphenyl)-2-(N-methylamino)-1,2,3,4-tetrahydronaphthalene

The cis-amine prepared in Example 11(d) above was dissolved in 300 ml of dry dimethylsulfoxide (DMSO). To this was added 15 g (.134 mol) of potassium t-butoxide portion-wise over 30 minutes. The mixture was stirred at room temeprature for an additional 2 hrs., then poured into 300 ml of 10% sodium bicarbonate. The resulting mixture was extracted three times with 300 ml of ether. The ether layer was washed three times with 300 ml of water, dried over $MgSO_4$, and evaporated to give 13.1 g of the title compound slightly contaminated with the cis-amine (<3%). This was characterized by NMR:

PPM

| cis | | trans | |
|---|---|---|---|
| 2.55 | (S, 3H) | 2.32 | (S, 3H) |
| 2.8—3.8 | (M, 5H) | 2.6—3.2 | (M, 5H) |
| 3.80 | (S, 3H) | 3.70 | (S, 3H) |
| 4.36 | (D, 1H, J=5Hz) | 3.82 | (D, 1H, J=Hz) |
| 6.5—7.3 | (M, 3H) | 6.5—7.5 | (M, 8H) |

(f) trans-1-(3-Methoxyphenyl)-2-(N-chloroacetyl-N-methylamino)-1,2,3,4-tetrahydronaphthalene

8.7 g of the trans compound of Example 11(e) above (.032 mol) was dissolved in 100 ml of methylene chloride. To this was added 4.9 g (.036 mol) of potassium carbonate in 30 ml of water followed by 2.9 ml (.036 mol) of chloroacetyl chloride added dropwise over 5 minutes with vigorous stirring. After 2 hrs., the mixture was diluted with 300 ml of ethyl acetate. The aqueous layer was extracted with 50 ml of ethyl acetate. The combined organic layers were washed with 50 ml of 1M HCl and with 50 ml of water, dried over $MgSO_4$ and evaporated to the title compound as a chromatographically and spectroscopically pure oil, 11.0 g, which was characterized by IR: 1655 $cm^{-1}$.

(g) trans-6,7,7a,8,9,13b-hexahydro-2-methoxy-7-methyl-5H-benzo[d]naphtho[2,1-b]azepine

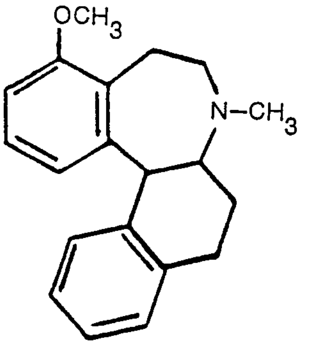

A

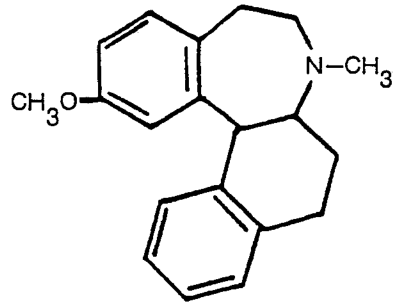

B

A solution of 2.8 g of the compound of Example 11(f) above in 400 ml of 60:40 ml of ethanol-water was irradiated with a 400W Hanovia lamp with Vycor filter for 3 hrs. at room temperature in a nitrogen atmosphere. The pH of the solution was adjusted to pH 8 with aqueous sodium carbonate and the solvent was evaporated. The residue was taken up in 300 ml of ethyl acetate, washed with 50 ml of water, dried over $MgSO_4$ and evaporated to a semi-solid. Chromatography on silica gel eluting with 30% ethyl acetated hexane gave 300 mg of the 9-methoxy derivative of formula A above and 370 mg of the desired 7-methoxy derivative of formula B above.

A portion of the 7-methoxy compound of formula 3 above was treated with excess borane-THF to give the title compound whose NMR and TLC behavior were identical with a fully characterized sample prepared by a different route.

Example 12

(a) 4-(3-Methoxyphenyl)-$\Delta^{3,4}$-chromene

A solution of 3-methoxyphenyl magnesium bromide was prepared by adding 27.7 g of m-bromoanisole to a suspension of magnesium turnings (3.6 g) in 150 ml of tetrahydrofuran (THF) dropwise over 1 hr. The mixture was heated at reflux for 2 hrs. The resulting solution was cooled to 5°C and a solution of 4-chromanone (20.0 g) in 50 ml of dry THF added over 2 hrs. at 15°C. The resulting mixture was stirred overnight at room temperature, cooled to 10°C, and treated with 1N HCl untl pH 7 was reached. The mixture was diluted with water, extracted twice with 500 ml of ether, and the combined extracts dried over $MgSO_4$. Filtration and evaporation gave 29.6 g of the title compound as an oil. NMR ($CDCl_3$); δ 3.81 (S, 3H), 4.80 (d, J=4Hz, 2H) 5.76 (t, J=4Hz, 1H), 6.7—7.4 (M, 8H).

(b) 4-(3-Methoxyphenyl)-chroman-2-one

m-Chloroperbenzoic acid (25.2 g, 80.85% pure) was added portionwise over 3 minutes to a stirred mixture of the title compound of Example 12(a) above (29.6 g) in 200 ml of $H_2O$, 23 g $NaHCO_3$ and 400 ml of $CH_2Cl_2$ at 5—10°C. Stirring was continued at 5—10°C for 3 hrs., the organic layer was separated, and the aqueous layer was extracted with 200 ml of $CH_2Cl_2$. The extracts were combined with the original organic layer and dried over $MgSO_4$. Filtration and evaporation of solvent gave a product which was dissolved in 200 ml of 1M NaOH in 3:1 ethanol-water and stirred at room temperature for 2 hrs. The pH was then brought to 9 with HCl, and most of the solvent was evaporated. The aqueous residue was extracted three times with 200 ml of portions of ether. The combined extracts were dried over $MgSO_4$, filtered and evaporated. The oily residue was taken up in 400 ml of toluene containing 0.5 g of p-toluene sulfonic acid and the solution was heated at reflux for 2 hrs. On cooling, the solution was washed with 100 ml of 0.5M $NaHCO_3$, dried over $MgSO_4$ and evaporated. The residue was chromatographed on silica gel eluting with toluene to give 10.3 g of the title compound as an oil. NMR ($CDCl_3$); δ 3.73 (S, 3H), 4.49 (Br.s, 2H), 4.70 (Br.s, 1H), 6.65—7.66 (M, 8H).

(c) 4-(m-Methoxyphenyl)-3-methylamino-$\Delta^{3,4}$-chromene

A stream of anhydrous methylamino gas was passed through a refluxing solution of 5.4 g of the title compound of Example 12(b) above in 200 ml of dry toluene for 1½ hr. until water evolution ceased. Cooling and evaporation gave 5.45 g of the title compound as an oil. NMR ($CDCl_3$) δ 2.63 (S, 3H), 3.77 (S, 3H), 4.83 (S, 2H), 6.40—7.62 (M, 8H).

(d) trans-4-(m-Methoxyphenyl)-3-methylamino-chroman

Sodium cyanoborohydride (1.27 g) was added to a mixture of the title compound of Example 12(c) above (5.4 g) in 400 ml of absolute alcohol followed by addition of 1.2 ml of glacial acetic acid. After stirring overnight at room temperature under a nitrogen atmosphere, 60 ml of 1M HCl was added and stirring continued for 30 minutes. The bulk of the solvent was then evaporated, and the residue partitioned between 300 ml ether and 300 ml water. The aqueous layer was separated and washed with 100 ml of ether. The pH adjusted to 8—10 with solid KOH. This mixture was then extracted three times with 300 ml portions

of ether, which were combined, dried, and evaporated to give 4.35 g an oily product.

4.0 g of this last material was dissolved in a mixture of 100 ml of dimethylsulfoxide (DMSO) and 25 ml of dimethylformamide (DMF), cooled to about 0°C, and potassium t-butoxide (5.0 g) was added portion wise over 5 more minutes. The mixture was then diluted with 100 ml of ice water followed by 5.0 g solid $NaHCO_3$. The resulting mixture was then poured into 400 ml of ether, and the organic layer separated. The aqueous layer was extracted with 400 ml ether, combined with the initial ether phase and washed twice with 100 ml portions of water. Drying over $MgSO_4$, filtration, and evaporation of solvent gave 3.9 g of the title compound as an oil. NMR Spectrum ($CDCl_3$), δ 2.48 (S, 3H), 3.05 (M, 1H), 3.77 (S, 3H), 3.96 (dd, 1H, J=7Hz, 11Hz), 4.26 (dd, 1H, J=3Hz, 11Hz), 6.65—7.30 (M, 8H).

(e) trans-3-[N-(2,2-Diethoxyethyl)-N-methylamino]-4-(3-methoxyphenyl)-chromone

A mixture of the title compound of Example 12(d) above (3.5 g), bromoacetaldehyde diethylacetal (4.0 ml), KI (0.33 g), $K_2CO_3$ (4.0 g) and dry dimethylformamide (150 ml) was heated at 155°C under a nitrogen atmosphere for 10 hrs, then allowed to stand at room temperature overnight. The mixture was then diluted with 700 ml of ether and washed three times with 150 ml portions of water. The organic layer was dried over $MgSO_4$, filtered, and evaporated in high vacuum to give 5.1 g the title compound as an oil. NMR Spectrum ($CDCl_3$): δ 1.11 (M, 6H), 2.43 (S, 3H), 2.69 (d, J=6Hz, 2H), 3.11 (M, 1H), 3.3—3.7 (M, 4h), 3.76 (S, 3H), 4.05—4.30 (M, 3H), 4.35 (t, 1H, J=6Hz), 6.65—6.90 (M, 6H), 7.05—7.25 (M, 2H).

(f) trans-6,6a,7,8,9,13b-Hexahydro-12-methoxy-7-methyl-[1]benzopyrano[4,3-a][3]benzazepine

To 250 ml of 18N sulfuric acid at 0°C was added 5.0 g of the title compound of Example 12(e) above. The heterogeneous mixture was stirred vigorously while coming to room temperature overnight. The now homogeneous mixture was poured over crushed ice. With continuous vigorous stirring and cooling via an ice bath, the pH was raised to 8—10 by slow addition of 50% NaOH over 3 hrs. The mixture was extracted three times, with 500 ml of ethyl acetate, dried over magnesium sulfate, and evaporated to give 3.6 g crude oil. This material was dissolved in 200 ml of ethyl acetate and hydrogenated over 5% palladium on carbon at 344.7 kPa (50 psi). After filtration, the mixture was separated by HPLC on silica gel eluting with ethyl acetate to give 219 mg of the title compound. NMR ($CDCl_3$): δ 2.45 (S, 3H), 2.49—2.98 (M, 4H), 3.14 (M, 1H), 3.59 (S, 3H), 3.7 (dd, J=10Hz, 11Hz, 1H), 4.39 (cd, 1H, J=10Hz, 5Hz), 4.74 (d, 1H, J=7.6Hz), 6.02 (d, 1H, J=1.6Hz), 6.56 (dd, 1H, J=8Hz, 2.6Hz), 6.83—7.31 (M, 5H).

Example 13

trans-6,6a,7,8,9,13b-Hexahydro-7-methyl-[1]benzopyrano[4,3-a][3]benzazepin-12-ol

A solution of the title compound from Example 12(f) above (330 mg) in 15 ml of $CH_2Cl_2$ was cooled to −78°C and $BBr_3$ (132 μl) added dropwise over 1 minute. The mixture was allowed to come to room temperature and was stirred for 23 hrs. under nitrogen. Methanol (5 ml) were then added and the mixture stirred for 10 minutes, after which it was evaporated to dryness. The methanol treatment was repeated, after which the mixture was evaporated in high vacuum to give 400 mg solids. These solids were dissolved in ethanol and the solution treated with decolorizing carbon at reflux. Filtration and evaporation gave 240 mg of the title compound, m.p. 223—226°C.

By application of the above-described techniques and related procedures known to those skilled in the art, the compounds listed in Table II below may also be synthesized.

TABLE II

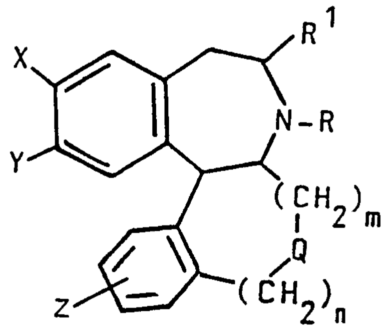

Ia

| Q | n | m | X | Y | Z | $R^1$ | R |
|---|---|---|---|---|---|---|---|
| $CH_2$ | 0 | 0 | $OCH_3$ | OH | H | H | $CH_3$ |
| $CH_2$ | 1 | 1 | Cl | OH | H | H | $CH_3$ |
| $CH_2$ | 1 | 1 | $OCH_3$ | OH | H | H | $CH_3$ |
| $CH_2$ | 1 | 1 | H | OH | H | H | $CH_3$ |
| S | 0 | 1 | Cl | OH | H | H | $CH_3$ |
| S | 0 | 1 | $OCH_3$ | OH | H | H | $CH_3$ |
| S | 0 | 1 | $CH_3$ | OH | H | H | $CH_3$ |
| S | 0 | 1 | H | OH | H | H | $CH_3$ |
| O | 0 | 1 | Cl | OH | H | H | $CH_3$ |
| O | 0 | 1 | $OCH_3$ | OH | H | H | $CH_3$ |
| O | 0 | 1 | H | OH | H | H | $CH_3$ |
| O | 0 | 1 | $CH_3$ | OH | H | H | $CH_3$ |
| $CH_2$ | 1 | 0 | Cl | OH | H | H | $CH_3$ |

| Q | n | m | X | Y | Z | $R^1$ | R |
|---|---|---|---|---|---|---|---|
| $CH_2$ | 1 | 0 | $OCH_3$ | OH | H | H | $CH_3$ |
| $CH_2$ | 1 | 0 | Cl | OH | H | H | H |
| $CH_2$ | 1 | 0 | H | $NH_2$ | H | H | $CH_3$ |
| $CH_2$ | 1 | 0 | $CH_3$ | $NH_2$ | H | H | $CH_3$ |
| $CH_2$ | 1 | 0 | Cl | $NH_2$ | H | H | $CH_3$ |
| $CH_2$ | 1 | 0 | H | OH | H | H | $CH_3$ |
| $CH_2$ | 1 | 0 | $CH_3$ | OH | H | H | $CH_3$ |
| $CH_2$ | 1 | 0 | Cl | * | H | H | $CH_3$ |
| $CH_2$ | 1 | 0 | $CH_3O$ | * | H | H | $CH_3$ |
| $CH_2$ | 1 | 0 | $CH_3O$ | OH | H | $CH_3$ | $CH_3$ |
| $CH_2$ | 1 | 0 | H | OH | 11-Cl | H | $CH_3$ |

Note: * = $-OCON(CH_3)_2$

**Claims**

1. A compound having the structural formula I, including isomers and pharmaceutically acceptable salts thereof,

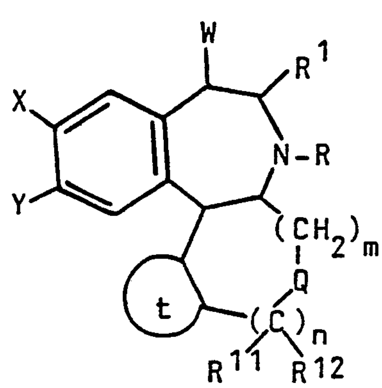

wherein:

R is hydrogen, $C_1$—$C_6$-alkyl, —$CH_2CH=CH_2$ or

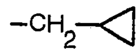 ;

$R^1$, $R^{11}$ and $R^{12}$ are the same or different and each is hydrogen or $C_1$—$C_6$-alkyl;

Q is methylene, —O— or —S—;

m and n are independently variable and may each have a value of 0, 1 or 2, with the provisos that the sum of m and n is not greater than 3, that m may not equal zero when Q is —O— or —S—, and that when Q is —$CH_2$— m and n cannot both be zero, unless W is H, X is $OCH_3$, Y is OH, Z is H, $R^1$ is H and R is $CH_3$;

X is hydrogen, F, Cl, Br, I, $C_1$—$C_6$-alkyl, $C_1$—$C_6$-alkylthio, $C_1$—$C_6$-alkylsulfinyl, $C_1$—$C_6$-alkylsulfonyl, hydroxy, $C_1$—$C_6$-alkoxy or trifluoromethyl;

Y is hydrogen, hydroxy, $C_1$—$C_6$-alkoxy,

$$-O\overset{O}{\overset{\|}{C}}NR^2R^3, \quad -O\overset{O}{\overset{\|}{C}}-R^9, \quad -NR^1_2, \quad -NH\overset{O}{\overset{\|}{C}}R^1 \text{ or } -O\overset{O}{\overset{\|}{P}}(OH)OR^1;$$

where

$R^1$ is as defined above;

W is hydrogen, hydroxy or $C_1$—$C_6$-alkoxy;

ring

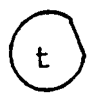

represents a fused thiophene or fused benzene ring said fused benzene ring optionally being substituted with a substituent 2 as defined below;

$R^2$ and $R^3$ are independently hydrogen (provided that both are not hydrogen), $C_1$—$C_6$-alkyl, phenyl-$C_1$—$C_6$-alkyl, cyclo-$C_3$—$C_7$-alkyl, phenyl or phenyl monosubstituted by $C_1$—$C_6$-alkyl, hydroxy, $C_1$—$C_6$-alkoxy, F, Cl, Br, I or trifluormethyl, hydroxy-$C_1$—$C_6$-alkyl, or $C_1$—$C_6$-alkoxy-$C_1$—$C_6$-alkyl;

in addition, when one of $R^2$ and $R^3$ is as defined above, the other may be —$R^4NR^5R^6$ {wherein $R^4$ is $C_1$—$C_6$-alkanediyl, $R^5$ is hydrogen or $C_1$—$C_6$-alkyl and $R^6$ is $C_1$—$C_6$-alkyl, or $R^5$ and $R^6$ together with the nitrogen atom form a 1-azetidinyl, 1-pyrrolidinyl, 1-piperidinyl, 1-(4-($C_1$—$C_6$-)alkylpiperazinyl), 4-morpholinyl or 1-hexahydrozepinyl group},

in further addition, $R^2$ and $R^3$ together with the nitrogen atom may form a 1-azetidinyl, 1-pyrrolidinyl, 1-piperidinyl, 4-morpholinyl, 1-(4-($C_1$—$C_6$)-alkylpiperazinyl), 1-(4-($C_1$—$C_6$)-alkoxy-($C_1$—$C_6$)-alkylpiperazinyl), 1-(4-hydroxy-($C_1$—$C_6$)-alkylpiperazinyl), 1-(3-hydroxyazetidinyl), 1-(3-($C_1$—$C_6$)-alkoxyazetidinyl), 1-(3-hydroxy-pyrrolidinyl), 1-(3-($C_1$—$C_6$)-alkoxypyrrolidinyl), 1-(3- or 4-hydroxypiperidinyl), 1-($C_1$—$C_6$)-(3- or 4-($C_1$—$C_6$)-alkoxypiperidinyl), 1-(4-oxopiperidinyl) or 1-(3-oxopyrrolidinyl) ring;

in still further addition, when $R^2$ is hydrogen, $R^3$ may be —$CHR^7CO_2R^8$, wherein $R^7$ and $R^8$ are independently hydrogen, $C_1$—$C_6$-alkyl or phenyl-$C_1$—$C_6$-alkyl;

$R^9$ is $C_1$—$C_6$-alkyl, phenyl-$C_1$—$C_6$-alkyl, phenyl or phenyl monosubstituted by $C_1$—$C_6$-alkyl, hydroxy, $C_1$—$C_6$-alkoxy, F, Cl, Br, I or trifluormethyl, $C_1$—$C_6$-alkoxy-$C_1$—$C_6$-alkyl, phenyloxy-$C_1$—$C_6$-alkyl, phenyl-$C_1$—$C_6$-alkoxy-$C_1$—$C_6$-alkyl, cyclo-$C_3$—$C_7$-alkyl-$C_1$—$C_6$-alkyl, $C_1$—$C_6$-alkoxycarbonyl-$C_1$—$C_6$-alkyl, cyclo-$C_3$—$C_7$-alkyl, 1-adamantyl, cyclo-$C_3$—$C_7$-alkoxy-$C_1$—$C_6$-alkyl, $C_1$—$C_6$-alkoxy, phenyl-$C_1$—$C_6$-alkoxy, cyclo-$C_1$—$C_6$-alkoxy, phenoxy or phenoxy monosubstituted by $C_1$—$C_6$-alkyl, hydroxy, $C_1$—$C_6$-alkoxy, F, Cl, Br, I or trifluormethyl or —$CHR^7NHR^8$ {wherein $R^7$ and $R^8$ are as defined above}; and

Z is X as defined above, amino, $C_1$—$C_6$-alkylamino or

$$-NH\overset{O}{\overset{\|}{C}}R^{10}$$

{wherein $R^{10}$ is hydrogen, $C_1$—$C_6$-alkyl or phenyl or phenyl monosubstituted by $C_1$—$C_6$-alkyl, hydroxy, $C_1$—$C_6$-alkoxy, F, Cl, Br, I or trifluormethyl}.

2. A compound as defined in claim 1 wherein W, $R^{11}$ and $R^{12}$ are all hydrogen.

3. A compound as defined in claim 1 or claim 2 wherein

Y is hydroxy;

$$-O-\overset{O}{\overset{\|}{C}}NR^2R^3,$$

where $R^2$ and $R^3$ are both alkyl or one of $R^2$ and $R^3$ is hydrogen and the other is alkyl; —$NHR^1$ where $R^1$ is hydrogen or methyl;

$$NH\overset{\overset{O}{\|}}{C}R^1$$

wherein $R^1$ is hydrogen or methyl; or

$$-O\overset{\overset{O}{\|}}{C}R^9$$

wherein $R^9$ is as defined in claim 1;

X is hydrogen, $C_1$—$C_6$-alkyl, F, Cl, Br, I or $C_1$—$C_6$-alkoxy;

Z is hydrogen, F, Cl, Br, I, $C_1$—$C_6$-alkyl, hydroxy or $C_1$—$C_6$-alkoxy;

R is methyl; and,

$R^1$ is hydrogen or methyl.

4. A compound as defined in claim 1 or claim 2 wherein

Y is hydroxy, amino,

$$-O\overset{\overset{O}{\|}}{C}R^9,\ O\overset{\overset{O}{\|}}{C}N(CH_3)_2$$

or —$NHCH_3$ wherein $R^9$ is as defined in claim 1;

ring

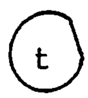

is a fused benzene ring;

Z is hydrogen, F, Cl, Br, I, $C_1$—$C_6$-alkyl or —$OR^1$ where $R^1$ is hydrogen or $C_1$—$C_6$-alkyl;

X is hydrogen, methyl, methoxy, chloro or bromo;

R is methyl;

$R^1$ is hydrogen;

Q is methylene;

and the sum of m and n equals 1.

5. A compound as defined in claim 1 which is:

(1) 6,7,7a,8,9,13b-hexahydro-2-hydroxy-3-methoxy-7-methyl-5*H*-benzo[d]naphtho[2,1-b]azepine or a pharmaceutically acceptable salt thereof;

(2) 6,7,7a,8,9,13b-hexahydro-2-hydroxy-7-methyl-5*H*-benzo[d]naphtho[2,1-b]azepine or a pharmaceutically acceptable salt thereof;

(3) 6,7,7a,8,9,13b-hexahydro-3-chloro-2-hydroxy-7-methyl-5*H*-benzo[d]naphtho[2,1-b]azepine or a pharmaceutically acceptable salt thereof;

(4) 6,7,7a,8,9,13b-hexahydro-2-hydroxy-3-methyl-7-methyl-5*H*-benzo[d]naphtho[2,1-b]azepine or a pharmaceutically acceptable salt thereof;

(5) 6,7,7a,8,9,13b-hexahydro-2-amino-7-methyl-5*H*-benzo[d]naphtho[2,1-b]azepine or a pharmaceutically acceptable salt thereof;

(6) 6,7,7a,8,9,13b-hexahydro-2-amino-3-chloro-7-methyl-5*H*-benzo[d]naphtho[2,1-b]azepine or a pharmaceutically acceptable salt thereof;

(7) 6,7,7a,8,9,13b-hexahydro-2-amino-3-methyl-7-methyl-5*H*-benzo[d]naphtho[2,1-b]azepine, or a pharmaceutically acceptable salt thereof;

(8) 6,6a,7,8,9,13b-hexahydro-12-methoxy-7-methyl-[1]benzopyrano[4,3-a][3]benzazepine, or a pharmaceutically acceptable salt thereof;

(9) 6,6a,7,8,9,13b-hexahydro-3-hydroxy-2-methoxy-7-methyl-5*H*-benzo[d]naphtho[2,1-b]azepine, or a pharmaceutically acceptable salt thereof;

(10) 2-hydroxy-3-methoxy-7-methyl-5,6,7,7a,8,9,10,14b-octahydro-benzo[d]benzo[3,4]cyclohepta[1,2-b]azepine, or a pharmaceutically acceptable salt thereof; or

(11) 3-hydroxy-2-methoxy-7-methyl-5,6,7,7a,8,9,10,14b-octahydro-benzo[d]benzo[3,4]cyclohepta[1,2-b]azepine, or a pharmaceutically acceptable salt thereof.

6. The compound of claim 1 which is:

trans-6,7,7a,8,9,13b-hexahydro-3-chloro-2-hydroxy-7-methyl-5H-benzo[d]naphtho[2,1-d]azepine or a pharmaceutically acceptable salt thereof; or,

(−)-trans-6,7,7a,8,9,13b-hexahydro-3-chloro-2-hydroxy-7-methyl-5*H*-benzo[d]naphtho[2,1-d]azepine, or a pharmaceutically acceptable salt thereof; or,

(+)-trans-6,7,7a,8,9,13b-hexahydro-3-chloro-2-hydroxy-7-methyl-5H-benzo[d]naphtho[2,1-d]azepine or a pharmaceutically acceptable salt thereof; or,

trans-6,7,7a,8,9,13b-hexahydro-2-hydroxy-3-methoxy-7-methyl-5H-benzo[d]naphtho[2,1-d]azepine or a pharmaceutically acceptable salt thereof; or

trans-6,6a,7,8,9,13b-hexahydro-12-hydroxy-7-methyl-[1]benzopyrano[4,3-a][3]benzazepine or a pharmaceutically acceptable salt thereof.

7. A pharmaceutical composition which comprises a compound as defined in any one of claims 1—6 in combination with a pharmaceutically acceptable carrier.

8. The use of a composition of any one of claims 1—6 for the preparation of a pharmaceutical composition useful in the treatment of psychoses, in the treatment of pain, in the treatment of depression, in the treatment of depression and/or as a renal vasodilator.

9. A compound of the formula XI or XIV

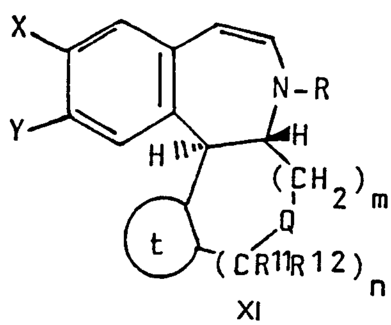

XI

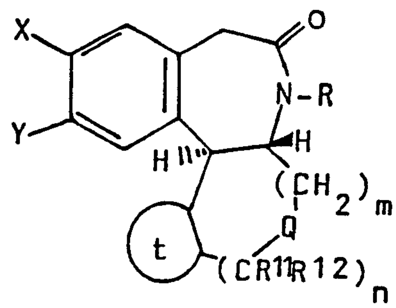

XIV

wherein R, $R^1$ to $R^{12}$, Q, m, n, W, X, Y, Z and ⓣ are defined according to claim 1.

10. A process for producing a compound having the structural formula I

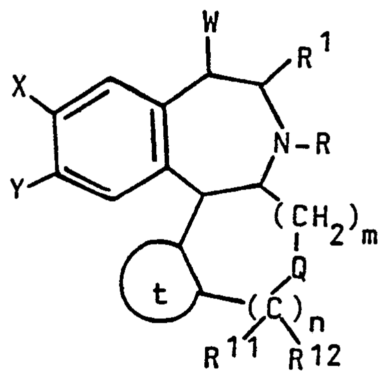

wherein R, $R^1$ to $R^{12}$, Q, m, n, W, X, Y, Z and ⓣ are defined according to claim 1, characterized by;

(a) intramolecular condensation of a compound of the formula

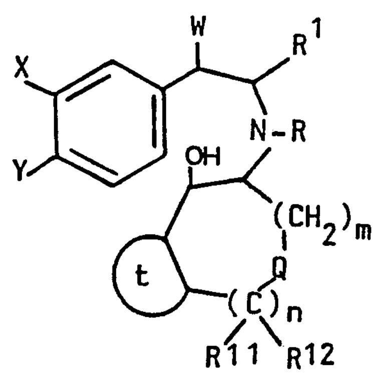

II

(b) by reacting a compound of the formula

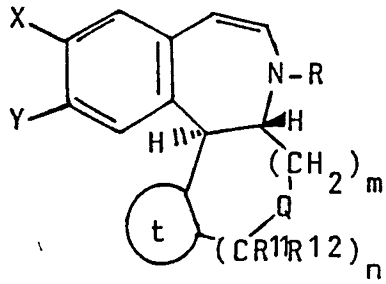

XI

with a reducing agent;

(c) contacting a compound of the formula

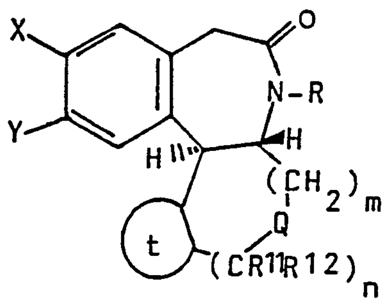

XIV

with a reducing agent.

11. A method for making a pharmaceutical composition according to claim 7 comprising mixing a compound of formula I with a pharmaceutically acceptable carrier.

12. A compound as defined in any of claims 1—3 above wherein:
Q is methylene;
m and n are both zero;
X is methoxy;
Y is hydroxy;
Z is hydrogen;
$R^1$ is hydrogen; and
R is methyl.

## Patentansprüche

1. Verbindung der Strukturformel I, einschließlich der Isomeren und der pharmazeutisch annehmbaren Salze derselben

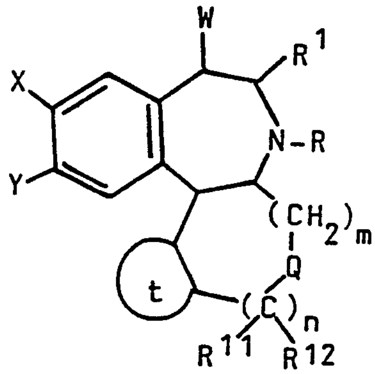

worin

R Wasserstoff, $C_1$—$C_6$-Alkyl, $—CH_2CH{=}CH_2$ oder

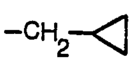 ;

ist;

$R^1$, $R^{11}$ und $R^{12}$ gleich oder verschieden sind und jeweils Wasserstoff oder $C_1$—$C_6$-Alkyl sind;

Q Methylen, —O— oder —S— ist;

m und n unabhängig voneinander variabel sind und jeweils einen Wert von 0, 1 oder 2 haben können, mit den Maßgaben, daß die Summe von m und n nicht größer als 3 ist, daß m nicht gleich null sein darf, wenn Q —O— oder —S— ist und daß, wenn Q —$CH_2$— ist, m und n nicht beide 0 sein können, sofern nicht W H ist, X $OCH_3$ ist Y OH ist, Z H ist, $R^1$ H ist und R $CH_3$ ist;

X Wasserstoff, F, Cl, Br, I, $C_1$—$C_6$-Alkyl, $C_1$—$C_6$-Alkylthio, $C_1$—$C_6$-Alkylsulfinyl, $C_1$—$C_6$-Alkylsulfonyl, Hydroxy, $C_1$—$C_6$-Alkoxy oder Trifluoromethyl ist;

Y Wasserstoff, Hydroxy, $C_1$—$C_6$-Alkoxy,

$$-O\overset{O}{\overset{\|}{C}}NR^2R^3, \quad -O\overset{O}{\overset{\|}{C}}-R^9, \quad -NR^1_2, \quad -NH\overset{O}{\overset{\|}{C}}R^1 \text{ oder } -O\overset{O}{\overset{\|}{P}}(OH)OR^1;$$

ist, worin $R^1$ die im Vorstehenden angegebenen Bedeutungen hat;

W Wasserstoff, Hydroxy oder $C_1$—$C_6$-Alkoxy ist;

der Ring

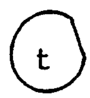

einen anellierten Thiophen-Ring oder einen anellierten Benzol-Ring bezeichnet, wobei der anellierte Benzol-Ring gegebenenfalls mit einem Substituenten Z substituiert ist, wie er im Folgenden definiert ist,

$R^2$ und $R^3$ unabhängig voneinander Wasserstoff (vorausgesetzt daß nicht beide Wasserstoff sind), $C_1$—$C_6$-Alkyl, Phenyl-$C_1$—$C_6$-alkyl, Cyclo-$C_3$—$C_7$-alkyl, Phenyl oder Phenyl, das durch $C_1$—$C_6$-Alkyl, Hydroxy, $C_1$—$C_6$-Alkoxy, F, Cl, Br, I oder Trifluoromethyl monosubstituiert ist, Hydroxy-$C_1$—$C_6$-alkyl oder $C_1$—$C_6$-Alkoxy-$C_1$—$C_6$-alkyl ist;

zusätzlich, wenn einer der Substituenten $R^2$ und $R^3$ eine der im Vorstehenden angegebenen Bedeutungen hat, der andere —$R^4NR^5R^6$ sein kann {worin $R^4$ $C_1$—$C_6$-Alkandiyl ist, $R^5$ Wasserstoff oder $C_1$—$C_6$-Alkyl ist und $R^6$ $C_1$—$C_6$-Alkyl ist oder $R^5$ und $R^6$ zusammen mit dem Stickstoff-Atom eine 1-Azetidinyl-, 1-Pyrrolidinyl-, 1-Piperidinyl-, 1-[4-($C_1$—$C_6$)-Alkylpiperazinyl]-, 4-Morpholinyl- oder 1-Hexahydroazepinyl-Gruppe bilden},

weiterhin zusätzlich $R^2$ und $R^3$ zusammen mit dem Stickstoff-Atom einen 1-Azetidinyl-, 1-Pyrrolidinyl-, 1-Piperidinyl-, 4-Morpholinyl-, 1-[4-($C_1$—$C_6$)-Alkylpiperazinyl]-, 1-[4-($C_1$—$C_6$)-Alkoxy-($C_1$—$C_6$)-alkylpiperazinyl]-, 1-[4-Hydroxy-($C_1$—$C_6$)-alkylpiperazinyl]-, 1-(3-Hydroxyazetidinyl)-, 1-[3-($C_1$—$C_6$)-Alkoxyazetidinyl]-, 1-(3-Hydroxypyrrolidinyl)-, 1-[3-($C_1$—$C_6$)-Alkoxypyrrolidinyl-, 1-(3- oder 4-Hydroxypiperidinyl)-, 1-[3- oder 4-($C_1$—$C_6$)-Alkoxypiperidinyl]-, 1-(4-Oxopiperidinyl)- oder 1-(3-Oxopyrolidinyl)-Ring bilden können;

noch weiterhin zusätzlich dann, wenn $R^2$ Wasserstoff ist, $R^3$ —$CHR^7CO_2R^8$ sein kann, worin $R^7$ und $R^8$ unabhängig voneinander Wasserstoff, $C_1$—$C_6$-Alkyl, oder Phenyl-$C_1$—$C_6$-alkyl sind,

$R^9$ $C_1$—$C_6$-Alkyl, Phenyl-$C_1$—$C_6$-alkyl, Phenyl oder Phenyl, das durch $C_1$—$C_6$-Alkyl, Hydroxy, $C_1$—$C_6$-Alkoxy, F, Cl, Br, I oder Trifluoromethyl monosubstituiert ist, $C_1$—$C_6$-Alkoxy-$C_1$—$C_6$-alkyl, Phenyloxy-$C_1$—$C_6$-alkyl, Phenyl-$C_1$—$C_6$-alkoxy-$C_1$—$C_6$-alkyl, Cyclo-$C_3$—$C_7$-alkyl-$C_1$—$C_6$-alkyl, $C_1$—$C_6$-Alkoxycarbonyl-$C_1$—$C_6$-alkyl, Cyclo-$C_3$—$C_7$-alkyl, 1-Adamantyl, Cyclo-$C_3$—$C_7$-alkoxy-$C_1$—$C_6$-alkyl, $C_1$—$C_6$-Alkoxy, Phenyl-$C_1$—$C_6$-alkoxy, Cyclo-$C_3$—$C_7$-alkoxy, Phenoxy oder Phenoxy, das durch $C_1$—$C_6$-Alkyl, Hydroxy, $C_1$—$C_6$-Alkoxy, F, Cl, Br, I oder Trifluoromethyl monosubstituiert ist, oder —$CHR^7NHR^8$ sein kann (worin $R^7$ und $R^8$ die im Vorstehenden angegebenen Bedeutungen haben); und

Z X, wie es im Vorstehenden definiert ist, Amino, $C_1$—$C_6$-Alkylamino oder

$$-NH\overset{O}{\overset{\|}{C}}R^{10}$$

ist

(worin $R^{10}$ Wasserstoff, $C_1$—$C_6$-Alkyl oder Phenyl oder Phenyl, das durch $C_1$—$C_6$-Alkyl, Hydroxy, $C_1$—$C_6$-Alkoxy, F, Cl, Br, I oder Trifluoromethyl monosubstituiert ist).

2. Verbindung nach Anspruch 1, worin W, $R^{11}$ und $R^{12}$ sämtlich Wasserstoff sind.

3. Verbindung nach Anspruch 1 oder Anspruch 2, worin

Y Hydroxy,

$$-O-\overset{O}{\overset{\|}{C}}NR^2R^3,$$

worin $R^2$ und $R^3$ beide Alkyl sind oder einer der Substituenten $R^2$ und $R^3$ Wasserstoff ist und der andere

Alkyl ist, —$NHR^1$, worin $R^1$ Wasserstoff oder Methyl ist,

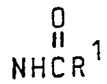

worin $R^1$ Wasserstoff oder Methyl ist, oder

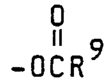

ist, worin $R^9$ die in Anspruch 1 angegebenen Bedeutungen hat;
X Wasserstoff, $C_1$—$C_6$-Alkyl, F, Cl, Br, I oder $C_1$—$C_6$-Alkoxy ist,
Z Wasserstoff, F, Cl, Br, I, $C_1$—$C_6$-Alkyl, Hydroxy oder $C_1$—$C_6$-Alkoxy ist,
R Methyl ist und
$R^1$ Wasserstoff oder Methyl ist.

4. Verbindung nach Anspruch 1 oder Anspruch 2, worin
Y Hydroxy, Amino,

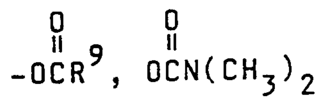

oder —$NHCH_3$ ist, worin $R^9$ die in Anspruch 1 angegebenen Bedeutungen hat;
der Ring

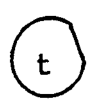

ein anellierter Benzol-Ring ist;
Z Wasserstoff, F, Cl, Br, I, $C_1$—$C_6$-Alkyl oder —$OR^1$ ist, worin $R^1$ Wasserstoff oder $C_1$—$C_6$-Alkyl ist;
X Wasserstoff, Methyl, Methoxy, Chloro oder Bromo ist;
R Methyl ist;
$R^1$ Wasserstoff ist;
Q Methylen ist; und
die Summe von m und n gleich 1 ist.

5. Verbindung nach Anspruch 1, die
(1) 6,7,7a,8,9,13b-Hexahydro-2-hydroxy-3-methoxy-7-methyl-5*H*-benzo[d]naphtho[2,1-b]azepin oder ein pharmazeutisch annehmbares Salz derselben,
(2) 6,7,7a,8,9,13b-Hexahydro-2-hydroxy-7-methyl-5*H*-benzo[d]naphtho[2,1-b]azepin oder ein pharmazeutisch annehmbares Salz derselben,
(3) 6,7,7a,8,9,13b-Hexahydro-3-chloro-2-hydroxy-7-methyl-5*H*-benzo[d]naphtho[2,1-b]azepin oder ein pharmazeutisch annehmbares Salz derselben,
(4) 6,7,7a,8,9,13b-Hexahydro-2-hydroxy-3-methyl-7-methyl-5*H*-benzo[d]naphtho[2,1-b]azepin oder ein pharmazeutisch annehmbares Salz derselben,
(5) 6,7,7a,8,9,13b-Hexahydro-2-amino-7-methyl-5*H*-benzo[d]naphtho[2,1-b]azepin oder ein pharmazeutisch annehmbares Salz derselben,
(6) 6,7,7a,8,9,13b-Hexahydro-2-amino-3-chloro-7-methyl-5*H*-benzo[d]naphtho[2,1-b]azepin oder ein pharmazeutisch annehmbares Salz derselben,
(7) 6,7,7a,8,9,13b-Hexahydro-2-amino-3-methyl-7-methyl-5*H*-benzo[d]naphtho[2,1-b]azepin oder ein pharmazeutisch annehmbares Salz derselben,
(8) 6,7,7a,8,9,13b-Hexahydro-12-methoxy-7-methyl-[1]benzopyrano[4,3-a][3]benzazepin oder ein pharmazeutisch annehmbares Salz derselben,
(9) 6,7,7a,8,9,13b-Hexahydro-3-hydroxy-2-methoxy-7-methyl-5*H*-benzo[d]naphtho[2,1-b]azepin oder ein pharmazeutisch annehmbares Salz derselben,
(10) 2-Hydroxy-3-methoxy-7-methyl-5,6,7,7a,8,9,10,14b-octahydro-benzo[d]benzo[3,4]cyclohepta[1,2-b]azepin oder ein pharmazeutisch annehmbares Salz derselben oder
(11) 3-Hydroxy-3-methoxy-7-methyl-5,6,7,7a,8,9,10,14b-octahydro-benzo[d]benzo[3,4]cyclohepta[1,2-b]azepin oder ein pharmazeutisch annehmbares Salz derselben
ist.

6. Verbindung nach Anspruch 1, die
trans-6,7,7a,8,9,13b-Hexahydro-3-chloro-2-hydroxy-7-methyl-5*H*-benzo[d]naphtho[2,1-b]azepin oder ein pharmazeutisch annehmbares Salz derselben oder

(−)-trans-6,7,7a,8,9,13b-Hexahydro-3-chloro-2-hydroxy-7-methyl-5*H*-benzo[d]naphtho[2,1-b]azepin oder ein pharmazeutisch annehmbares Salz derselben oder

(+)-trans-6,7,7a,8,9,13b-Hexahydro-3-chloro-2-hydroxy-7-methyl-5*H*-benzo[d]naphtho[2,1-b]azepin oder ein pharmazeutisch annehmbares Salz derselben oder

trans-6,7,7a,8,9,13b-Hexahydro-2-hydroxy-3-methoxy-7-methyl-5*H*-benzo[d]naphtho[2,1-b]azepin oder ein pharmazeutisch annehmbares Salz derselben oder

trans-6,7,7a,8,9,13b-Hexahydro-12-hydroxy-7-methyl-[1]benzopyrano[4,3-a][3]benzazepin oder ein pharmazeutisch annehmbares Salz derselben ist.

7. Pharmazeutische Zusammensetzung, umfassend eine Verbindung nach irgendeinem der Ansprüche 1 bis 6 in Kombination mit einem pharmazeutisch annehmbaren Träger.

8. Verwendung einer Zusammensetzung nach irgendeinem der Ansprüche 1 bis 6 zur Herstellung einer pharmazeutischen Zusammensetzung, die bei der Behandlung von Psychosen, bei der Behandlung von Schmerzen, bei der Behandlung von Depressionen, bei der Behandlung von Depressionen und/oder als renales gefäßerweiterndes Mittel von Nutzen ist.

9. Verbindung der Formel XI oder XIV

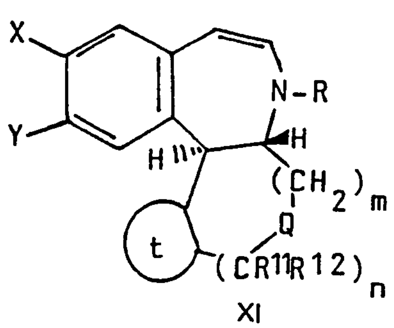

XI

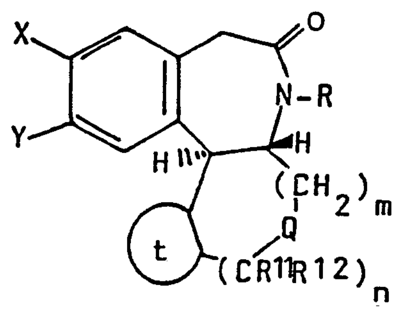

XIV

worin R, $R^1$ bis $R^{12}$, Q, m, n, W, X, Y, Z und

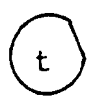

die in Anspruch 1 angegebenen Bedeutungen haben.

10. Verfahren zur Herstellung einer Verbindung der Strukturformel I

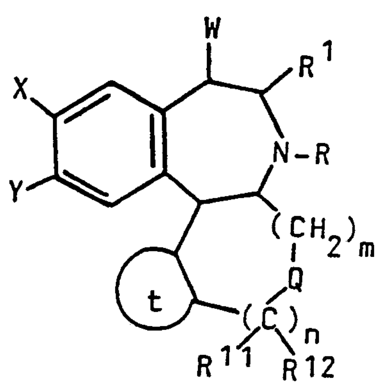

worin R, $R^1$ bis $R^{12}$, Q, m, n, W, X, Y, Z und

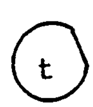

die in Anspruch 1 angegebenen Bedeugungen haben, gekennzeichnet durch

(a) intramolekulare Kondensation einer Verbindung der Formel

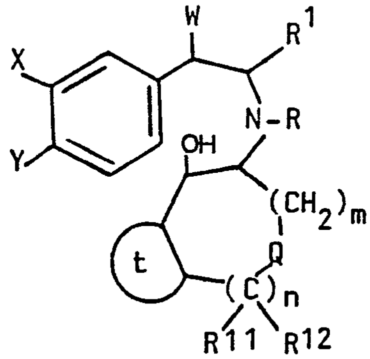

II

(b) Umsetzung einer Verbindung der Formel

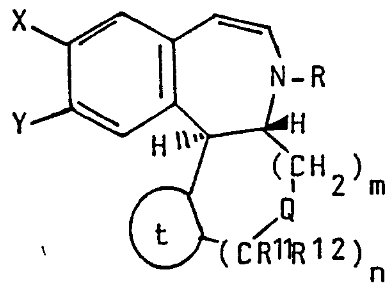

XI

mit einem Reduktionsmittel;

(c) In-Berührung-Bringen einer Verbindung der Formel

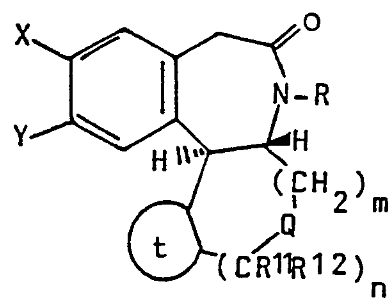

XIV

mit einem Reduktionsmittel.

11. Verfahren zur Herstellung einer pharmazeutischen Zusammensetzung nach Anspruch 7, umfassend das Vermischen einer Verbindung nach Anspruch 1 mit einem pharmazeutisch annehmbaren Träger.

12. Verbindung nach einem der vorstehenden Ansprüche 1 bis 3, worin
Q Methylen ist,
m und n beide null sind,
X Methoxy ist,
Y Hydroxy ist,
Z Wasserstoff ist,
$R^1$ Wasserstoff ist und
R Methyl ist.

**Revendications**

1. Composé ayant la formule de structure I, comprenant ses isomères et sels acceptables en pharmacie,

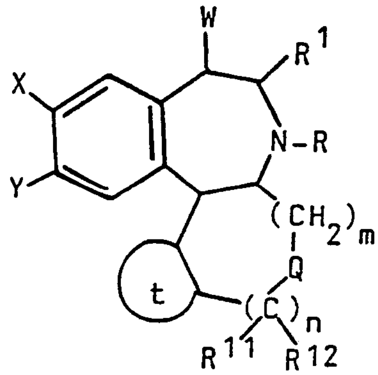

où:

R est hydrogène, alkyle($C_1$—$C_6$), —$CH_2CH$=$CH_2$ ou

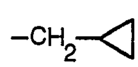 ;

$R^1$, $R^{11}$ et $R^{12}$ sont identiques ou différents et chacun est hydrogène ou alkyle ($C_1$—$C_6$);

Q est méthylène, —O— ou —S—;

m et n sont indépendamment variables et chacun peut avoir une valeur de 0, 1 ou 2, à condition que la somme de m et n ne dépasse pas 3, que m ne soit pas égal à zéro quand Q est —O— ou —S—, et que quand Q est —$CH_2$— m et n ne puissent être tous deux zéro, à moins que W soit H, X soit $OCH_3$, Y soit OH, Z soit H, $R^1$ soit H et R soit $CH_3$;

X est hydrogène, F, Cl, Br, I, alkyle($C_1$—$C_6$), alkylthio($C_1$—$C_6$), alkylsulfinyle($C_1$—$C_6$), alkylsulfonyle-($C_1$—$C_6$), hydroxy, alkoxy $C_1$—$C_6$ ou trifluorométhyle;

Y est hydrogène, hydroxy, alcoxy($C_1$—$C_6$),

$$-O\overset{O}{\overset{\|}{C}}NR^2R^3,\quad -O\overset{O}{\overset{\|}{C}}-R^9,\quad -NR^1_2,\quad -NH\overset{O}{\overset{\|}{C}}R^1 \text{ ou } -O\overset{O}{\overset{\|}{P}}(OH)OR^1;$$

où $R^1$ est tel que défini ci-dessus;

W est hydrogène, hydroxy ou alcoxy ($C_1$—$C_6$).

le noyau

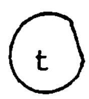

représente un noyau thiophène fondu ou benzène fondu, ledit noyau benzène fondu étant facultativement substitué par un substituant Z tel que défini ci-dessous;

$R^2$ et $R^3$ sont indépendamment hydrogène (à condition que tous deux ne soient pas hydrogène), alkyle-($C_1$—$C_6$), phénylalkyle ($C_1$—$C_6$), cycloalkyle($C_3$—$C_7$), phényle ou phényle monosubstitué par alkyle($C_1$—$C_6$), hydroxy, alcoxy($C_1$—$C_6$), F, Cl, Br, I ou trifluorométhyle, hydroxyalkyle($C_1$—$C_6$) ou alkoxy($C_1$—$C_6$)-alkyle-($C_1$—$C_6$);

de plus, lorsque l'un de $R^2$ et $R^3$ est tel que défini ci-dessus, l'autre peut être —$R^4NR^5R^6$ {où $R^4$ est alcanediyle($C_1$—$C_6$), $R^5$ est hydrogène ou alkyle($C_1$—$C_6$) et $R^6$ est alkyle($C_1$—$C_6$) ou $R^5$ et $R^6$ avec l'atome d'azote forment un groupe 1-azétidinyle, 1-pyrrolidinyle, 1-pipéridinyle, 1-(4-alkylpipérazinyle($C_1$—$C_6$)), 4-morpholinyle ou 1-hexahydroazépinyle},

de plus encore, $R^2$ et $R^3$, avec l'atome d'azote, peuvent former un noyau 1-azétidinyle, 1-pyrrolidinyle, 1-pipéridinyle, 4-morpholinyle, 1-(4-alkyle($C_1$—$C_6$)-pipérazinyle), 1-(4-alcoxy($C_1$—$C_6$)alkyle($C_1$—$C_6$)-pipérazinyle), 1-(4-hydroxyalkyle($C_1$—$C_6$)pipérazinyle), 1-(3-hydroxyazétidinyle), 1-(3-alcoxy($C_1$—$C_6$)-azétidinyle), 1-(3-hydroxypyrrolidinyle), 1-(3-alcoxy($C_1$—$C_6$)pyrrolidinyle), 1-(3- ou 4-hydroxypipéridinyle), 1-(3- ou 4-alcoxy($C_1$—$C_6$)pipéridinyle), 1-(4-oxopipéridinyle) ou 1-(3-oxopyrrolidinyle);

de plus encore, quand $R^2$ est hydrogène, $R^3$ peut être —$CHR^7CO_2R^8$, où $R^7$ et $R^8$ sont indépendamment

hydrogène, alkyle($C_1$—$C_6$) ou phényl- alkyle($C_1$—$C_6$);

$R^9$ est alkyle($C_1$—$C_6$), phénylalkyle($C_1$—$C_6$), phényle ou phényle mono-substitué par alkyle($C_1$—$C_6$), hydroxy, alcoxy($C_1$—$C_6$), F, Cl, Br, I ou trifluorométhyle, alcoxy($C_1$—$C_6$)alkyle($C_1$—$C_6$), phényloxyalkyle-($C_1$—$C_6$), phénylalcoxy($C_1$—$C_6$)alkyle($C_1$—$C_6$), cycloalkyl($C_3$—$C_7$)-alkyle($C_1$—$C_6$), alcoxy($C_1$—$C_6$)carbonyl-alkyle($C_1$—$C_6$), cycloalkyle($C_3$—$C_7$), l'adamantyle, cycloalcoxy($C_3$—$C_7$)-alkyle($C_1$—$C_6$), alcoxy($C_1$—$C_6$), phénylalcoxy($C_1$—$C_6$), cycloalcoxy($C_1$—$C_6$) phénoxy ou phénoxy mono-substitué par alkyle($C_1$—$C_6$), hydroxy, alcoxy($C_1$—$C_6$), F, Cl, Br, I ou trifluorométhyle ou bien —$CHR^7NHR^8$ {où $R^7$ et $R^8$ sont tels que définis ci-dessus}; et

Z est X tel que défini ci-dessus, amino, alkylamono($C_1$—$C_6$) ou

$$-NH\overset{O}{\overset{\|}{C}}R^{10}$$

{où $R^{10}$ est hydrogène, alkyle($C_1$—$C_6$) ou phényle ou phényle mono-substitué par alkyle($C_1$—$C_6$), hydroxy alcoxy($C_1$—$C_6$), F, Cl, Br, I ou trifluorométhyle}.

2. Composé tel que défini à la revendication 1, où W, $R^{11}$ et $R^{12}$ sont tous de l'hydrogène.

3. Composé tel que défini à la revendication 1 ou la revendication 2, où Y est hydroxy;

$$-O-\overset{O}{\overset{\|}{C}}NR^2R^3,$$

où $R^2$ et $R^3$ sont tous deux alkyles ou bien l'un de $R^2$ et $R^3$ est hydrogène et l'autre est alkyle; —$NHR^1$ où $R^1$ est hydrogène ou méthyle;

$$NH\overset{O}{\overset{\|}{C}}R^1$$

où $R^1$ est hydrogène ou méthyle; ou bien

$$-O\overset{O}{\overset{\|}{C}}R^9$$

où $R^9$ est tel que défini a la revendication 1;

X est hydrogène, alkyle($C_1$—$C_6$), F, Cl, Br, I ou alcoxy($C_1$—$C_6$),

Z est hydrogène, F, Cl, Br, I, alkyle($C_1$—$C_6$), hydroxy ou alcoxy($C_1$—$C_6$);

R est méthyle; et

$R^1$ est hydrogène ou méthyle.

4. Composé tel que défini à la revendication 1 ou à la revendication 2, où

Y est hydroxy, amino,

$$-O\overset{O}{\overset{\|}{C}}R^9,\ O\overset{O}{\overset{\|}{C}}N(CH_3)_2$$

ou —$NHCH_3$ ou $R^9$ est tel que défini à la revendication 1;

le noyau

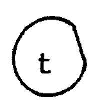

est un noyau benzène fondu;

Z est hydrogène, F, Cl, Br, I, alkyle($C_1$—$C_6$) ou —$OR^1$ où $R^1$ est hydrogène ou alkyle($C_1$—$C_6$);

X est hydrogène, méthyle, méthoxy, chloro ou bromo;

R est méthyle;

$R^1$ est hydrogène;

Q est méthylène;

et la somme de m et n est égale à 1.

5. Composé tel que défini à la revendication 1 qui est:

(1) 6,7,7a,8,9,13b-hexahydro-2-hydroxy-3-méthoxy-7-méthyl-5*H*-benzo[d]naphto[2,1-b]azépine ou son sel acceptable en pharmacie;

(2) 6,7,7a,8,9,13b-hexahydro-2-hydroxy-7-méthyl-5*H*-benzo[d]naphto[2,1-b]azépine ou son sel acceptable en pharmacie;

(3) 6,7,7a,8,9,13b-hexahydro-3-chloro-2-hydroxy-7-méthyl-5*H*-benzo[d]naphto[2,1-b]azépine ou son sel acceptable en pharmacie;

(4) 6,7,7a,8,9,13b-hexahydro-2-hydroxy-3-méthyl-7-méthyl-5*H*-benzo[d]naphto[2,1-b]azépine ou son sel acceptable en pharmacie;

(5) 6,7,7a,8,9,13b-hexahydro-2-amino-7-méthyl-5*H*-benzo[d]naphto[2,1-b]azépine ou son sel acceptable en pharmacie;

(6) 6,7,7a,8,9,13b-hexahydro-2-amino-3-chloro-7-méthyl-5*H*-benzo[d]naphto[2,1-b]azépine ou son sel acceptable en pharmacie;

(7) 6,7,7a,8,9,13b-hexahydro-2-amino-3-méthyl-7-méthyl-5*H*-benzo[d]naphto[2,1-b]azépine, ou son sel acceptable en pharmacie;

(8) 6,6a,7,8,9,13b-hexahydro-12-méthoxy-7-méthyl-[1]bezopyrano[4,3-a][3]benzazépine ou son sel acceptable en pharmacie;

(9) 6,6a,7,8,9,13b-hexahydro-3-hydroxy-2-méthoxy-7-méthyl-5*H*-benzo[d]naphto[2,1-b]azépine, ou son sel acceptable en pharmacie;

(10) 2-hydroxy-3-méthoxy-7-méthyl-5,6,7,7a,8,9,10,14b-octahydro-benzo[d]benzo[3,4]cyclohepta[1,2-b]azépine ou son sel acceptable en pharmacie; ou

(11) 3-hydroxy-3-méthoxy-7-méthyl-5,6,7,7a,8,9,10,14b-octahydro-benzo[d]benzo[3,4]cyclohepta[1,2-b]azépine, ou son sel acceptable en pharmacie.

6. Composé de la revendication 1 qui est:

trans-6,7,7a,8,9,13b-hexahydro-3-chloro-2-hydroxy-7-méthyl-5H-benzo[d]naphto[2,1-d]azépine ou son sel acceptable en pharmacie; ou

(−)-trans-6,7,7a,8,9,13b-hexahydro-3-chloro-2-hydroxy-7-méthyl-5*H*-benzo[d]naphto[2,1-d]azépine, ou son sel acceptable en pharmacie; ou

(+)-trans-6,7,7a,8,9,13b-hexahydro-3-chloro-2-hydroxy-7-méthyl-5H-benzo[d]naphto[2,1-b]azépine ou son sel acceptable en pharmacie; ou

trans-6,7,7a,8,9,13b-hexahydro-2-hydroxy-3-méthoxy-7-méthyl-5H-benzo[d]naphto[2,1-b]azépine ou son sel acceptable en pharmacie; ou

trans-6,6a,7,8,9,13b-hexahydro-12-hydroxy-7-méthyl-[1]benzopyrano[4,3-a][3]benzazépine ou son sel acceptable en pharmacie.

7. Composition pharmaceutique qui comprend un composé tel que défini selon l'une quelconque des revendications 1—6 en combinaison avec un véhicule acceptable en pharmacie.

8. Utilisation d'une composition selon l'une quelconque des revendications 1—6 pour la préparation d'une composition pharmaceutique utile dans le traitement des psychoses, dans le traitement de la douleur, dans le traitement de la dépression, dans le traitement de la dépression et/ou en tant que vasodilatateur rènal.

9. Composé de la formule XI ou XIV

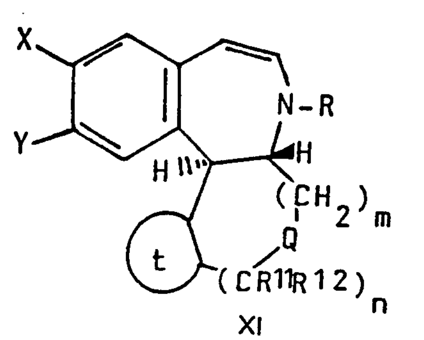

XI

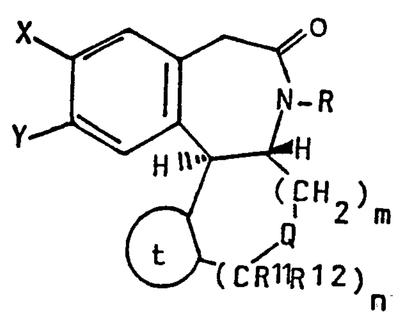

XIV

où R, $R^1$ à $R^{12}$, Q, m, n, W, X, Y, Z et ⓣ sont définis selon la revendication 1.

10. Procédé pour la production d'un composé ayant la formule de structure I

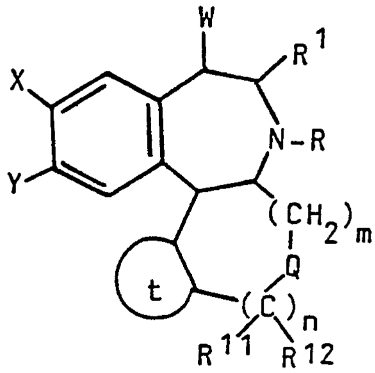

où R, $R^1$ à $R^{12}$, Q, m, n, W, X, Y, Z et ⓣ sont définis selon la revendication 1, caractérisé par

(a) la condensation intramoléculaire d'un composé de la formule

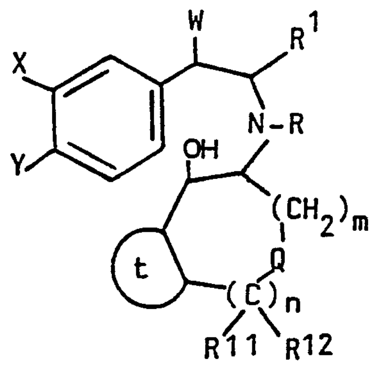

II

(b) par réaction d'un composé de la formule

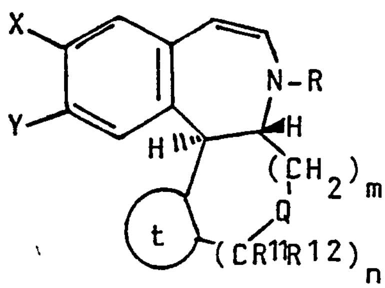

XI

avec un agent réducteur;

(c) la mise en contact d'un composé de la formule:

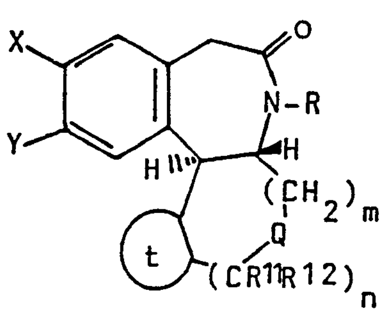

XIV

avec un agent réducteur.

11. Procédé de fabrication d'une composition pharmaceutique selon la revendication 7, comprenant le mélange d'un composé de la formule I avec un véhicule acceptable en pharmacie.

12. Composé tel que défini selon l'une quelconque des revendications 1—3 ci-dessus, où:
Q est méthylène;
m et n sont tous deux zéro;
X est méthoxy;
Y est hydroxy;
Z est hydrogène;
$R^1$ est hydrogène; et
R est méthyle.